# FASCICULE DE BREVET EUROPEEN

(11) **EP 1 383 926 B1**
(45) Date de publication et mention de la délivrance du brevet: **06.05.2015**
(21) Numéro de dépôt: 02740785.7
(22) Date de dépôt: 30.04.2002
(51) Int. Cl.: G01N 33/38, C12Q 1/68

(54) **METHODE DE DIAGNOSTIC PRENATAL SUR CELLULE FOETALE ISOLEE DU SANG MATERNEL**
PRÄNATALES DIAGNOSEVERFAHREN AN EINER ISOLIERTEN FÖTALEN ZELLE AUS DEM MÜTTERLICHEN BLUT
PRENATAL DIAGNOSIS METHOD ON ISOLATED FOETAL CELL OF MATERNAL BLOOD

(30) Priorité: 30.04.2001 FR 0105824
(43) Date de publication de la demande: 28.01.2004
(73) Titulaire: INSTITUT NATIONAL DE LA SANTE ET DE LA RECHERCHE MEDICALE (INSERM), 75654 Paris Cédex 13 (FR); ASSISTANCE PUBLIQUE - HOPITAUX DE PARIS, 75001 Paris (FR); UNIVERSITE RENE DESCARTES (PARIS V), 75270 Paris Cedex 06 (FR)
(72) Inventeur: PATERLINI-BRECHOT, Patrizia, F-75015 Paris (FR)
(74) Mandataire: Desaix, Anne
(86) Numéro de dépôt international: PCT/FR2002/001505
(87) Numéro de publication internationale: WO 2002/088736

(56) Documents cités:
- WO-A-99/15892
- US-A- 5 501 954
- US-A- 5 641 628
- VONA GIOVANNA ET AL: "Isolation by size of epithelial tumor cells: A new method for the immunomorphological and molecular characterization of circulating tumor cells." AMERICAN JOURNAL OF PATHOLOGY, vol. 156, no. 1, janvier 2000 (2000-01), pages 57-63, XP002186868 ISSN: 0002-9440 cité dans la demande
- BIANCHI DIANA W: "Fetal cells in the maternal circulation: Feasibility for prenatal diagnosis." BRITISH JOURNAL OF HAEMATOLOGY, vol. 105, no. 3, juin 1999 (1999-06), pages 574-583, XP002186869 ISSN: 0007-1048 cité dans la demande
- VONA GIOVANNA ET AL: "Enrichment, immunomorphological, and genetic characterization of fetal cells circulating in maternal blood." AMERICAN JOURNAL OF PATHOLOGY. UNITED STATES JAN 2002, vol. 160, no. 1, janvier 2002 (2002-01), pages 51-58, XP002236986 ISSN: 0002-9440
- A. SEKIZAWA ET AL: 'Analysis of HLA-DQ α sequences for prenatal diagnosis in single fetal cells from maternal blood' HUMAN GENETICS vol. 102, no. 4, 23 Avril 1998, pages 393 - 396, XP055001347 DOI: 10.1007/s004390050710 ISSN: 0340-6717
- WATANABE A ET AL: "PRENATAL DIAGNOSIS OF ORNITHINE TRANSCARBAMYLASE DEFICIENCY BY USING A SINGLE NUCLEATED ERYTHROCYTE FROM MATERNAL BLOOD", HUMAN GENETICS, SPRINGER, BERLIN, DE, vol. 102, no. 6, 1 June 1998 (1998-06-01), pages 611-615, XP001121711, ISSN: 0340-6717, DOI: DOI:10.1007/S004390050750

## Description

La présente invention est relative à une nouvelle méthode de diagnostic prénatal non-invasive mise en oeuvre à partir d'un échantillon de sang maternel. Ladite méthode permet le diagnostic prénatal sur cellules foetales, épithéliales, non apoptotiques isolées du sang maternel après leur enrichissement par filtration, leur analyse morphologique ou immunologique et génétique. Les avantages de cette méthode sont en particulier son innocuité pour la mère ou le foetus et la très grande sensibilité et spécificité du diagnostic. Elle permet la détection précoce d'une anomalie génétique ou chromosomique du foetus, d'une pathologie génétique ou infectieuse (virale, bactérienne ou parasitaire) du foetus, d'un génotype précis, et en particulier du sexe génétique du foetus.

Les méthodes de diagnostic prénatal visent notamment à obtenir des informations génétiques sur un foetus ou un embryon. La recherche d'informations génétiques sur un foetus consiste, soit à identifier la présence d'un allèle spécifique d'un gène donné ou d'une combinaison d'allèles sur une séquence donnée d'ADN foetal, soit à associer génétiquement un polymorphisme de l'ADN foetal à un allèle particulier. Une des applications majeures du diagnostic prénatal génétique concerne la détection des anomalies congénitales.

Les méthodes de diagnostic prénatal génétique utilisées en clinique comprennent essentiellement des techniques invasives comme l'amniocentèse, le prélèvement de villosités choriales, le prélèvement de sang foetal ou les biopsies tissulaires. L'ensemble de ces techniques implique que des échantillons sont obtenus directement du foetus ou indirectement de structures ovulaires. Compte tenu du caractère très invasif de ces méthodes, celles-ci sont susceptibles de complications pour la mère ou le foetus. Parmi ces complications, on citera dans le cas de l'amniocentèse, un risque d'infection, d'hémorragie foeto-maternelle avec une allo-immunisation possible, de pertes de liquide amniotiques ou de douleurs abdominales. Selon les études, le risque estimé de fausse-couche après un prélèvement par amniocentèse est de 0,2 à 2,1% supérieur à celui d'un groupe contrôle. Par conséquent, l'amniocentèse n'est proposée qu'aux femmes chez qui le risque d'avoir un enfant avec un handicap génétique excède celui de la fausse-couche iatrogène.

Afin de limiter l'utilisation des techniques invasives de diagnostic prénatal présentant les risques de complications mentionnés ci-dessus et étant généralement désagréables et/ou source de stress pour la mère, le développement de nouvelles méthodes non invasives constitue un enjeu majeur de l'obstétrique moderne.

Les cellules foetales circulantes dans le sang maternel constituent en particulier une source de matériel génétique potentiellement utilisable pour le diagnostic prénatal génétique (1, 2).

En effet, au cours de la grossesse, différents types cellulaires d'origine foetale traversent le placenta et circulent dans le sang maternel (1). Ces types cellulaires incluent les cellules lymphoïdes et érythroïdes, les précurseurs myéloïdes et les cellules épithéliales trophoblastiques (cytotrophoblastes et syncyciotrophoblastes).

Des méthodes d'analyse du génome de cellules foetales circulantes dans le sang maternel, en vue d'un diagnostic prénatal, sont décrites dans l'état de la technique mais celles-ci restent relativement limitées en terme de sensibilité et de spécificité du diagnostic (3, 4, 5, 6). L'intérêt du développement d'une méthode de diagnostic prénatal non-invasive et hautement spécifique résulte dans la possibilité de diminuer par un tel test la proportion de diagnostics invasifs indiqués chez les femmes enceintes et dont le résultat est finalement négatif. A titre d'exemple, dans le cas de la trisomie 21, qui concerne une femme sur 700, à l'heure actuelle en France, un diagnostic prénatal n'est offert que si l'âge de la mère est égal à 38 ans tandis qu'un test de dépistage biochimique capable de détecter 60% des trisomies 21 au prix d'un taux d'amniocentèse de 5% est proposé aux femmes plus jeunes. Cependant, encore 40% des cas de trisomie 21 ne sont pas détectés par les tests actuellement disponibles. La détection prénatale de la trisomie 21 sur cellules foetates isolées du plasma maternel en utilisant la technique FISH a récemment été décrite. Cette approche est intéressante mais les cellules foetales étant rares dans le plasma (1 sur 500 à 1 sur 2000) et incluant souvent des cellules apoptotiques, un diagnostic fiable nécessiterait la mise en oeuvre de la méthode sur un très grand nombre de cellules, rendant impossible une application en routine. De plus, les cellules foetales euploïdes ne peuvent être identifiées par cette approche.

Une des limitations de ces approches provient du fait que les cellules foetales circulantes dans le sang sont présentes en très faible concentration. Des études fondées sur la détection par PCR du chromosome Y sur des échantillons de sang sans sélection préalable ont permis d'évaluer le nombre moyen de cellules foetales à environ 1 cellule par millilitre de sang (7). D'autre part, il a été montré que des cellules foetales d'origine myeloïde ou lymphoïde (CD34 ou CD38 positives) sont encore présentes dans le sang maternel jusqu'à 27 ans après une grossesse ou une fausse-couche (8). Leur isolement ne s'associe donc pas à la certitude qu'elles dérivent de la grossesse en cours. On conçoit donc aisément les difficultés techniques de développement d'un diagnostic prénatal sur sang maternel utilisable en routine et la nécessité de concentrer les cellules foetales épithéliales (trophoblastiques) qui sont détruites après chaque grossesse et qui permettent d'analyser le génome fatal de la grossesse en cours.

Plusieurs auteurs ont cependant proposé d'améliorer la sensibilité de la détection des cellules foetales dans le sang maternel par l'enrichissement de la population cellulaire contenue dans le sang maternel en cellules foetales ou par la séparation des cellules foetales contenues dans le sang maternel (pour revue 1, 2).

En particulier, l'état de la technique comprend l'enseignement de procédés de séparation des cellules foetales circulantes fondées sur leurs propriétés d'affinité vis-à-vis de certains anticorps.

Il est par exemple décrit dans la demande de brevet WO 97/42503 (BIOCOM S.A.) un procédé d'immunoséparation magnétique de cellules foetales consistant à fixer les cellules foetales sur des billes paramagnétiques sur lesquelles ont été fixés des anticorps dirigés contre des antigènes exprimés à la surface des cellules foetales. Il est encore décrit dans le brevet WO90/06509 (Flinders Technologies Pty, Ltd) une méthode d'isolement de cellules trophoblastiques pour le diagnostic prénatal à l'aide d'anticorps reconnaissant des antigènes exprimés à la surface des cellules trophoblastiques. Cependant, aucun anticorps spécifique exclusivement des cellules foetales n'a été mis en évidence à l'heure actuelle. Ce point est important car le diagnostic prénatal doit être effectué sur une population cellulaire foetale « pure ».

A titre d'exemples de méthodes d'enrichissement d'une population de cellules issues de sang maternel en cellules foetales, on citera en particulier les brevets US 5,714,325 (New England Medical Center Hospitals), US 5,580,724 (Board of Regents, The University of Texas System), et US 5,646,004 (Activated Cell Therapy, Inc.).

Le brevet US 5,714,325 enseigne une méthode d'enrichissement en granulocytes foetaux par séparation des granulocytes foetaux des cellules maternelles à l'aide d'anticorps reconnaissant des antigènes exprimés à la surface des granulocytes foetaux, en particulier à l'aide de la combinaison d'un anticorps anti-CD71 (récepteur de la transferrine) et d'un anticorps antiglycophorine A.

Le brevet US 5,580,724 décrit une autre méthode d'enrichissement en cellules foetales d'une population de cellules issues de sang maternel par stimulation *in vitro* de la prolifération des cellules foetales à l'aide de facteurs de croissance spécifiques.

L'enrichissement en cellules foetales peut aussi être réalisé par centrifugation d'un échantillon de sang dans une solution avec gradient de densité (PERCOLL, FICOLL, albumine, sucrose ou dextran par exemple) comme décrit dans le brevet US 5,646,004.

Cependant, il s'avère que ces protocoles comprenant plusieurs étapes d'isolement peuvent endommager les cellules foetales et donner lieu à des pertes cellulaires significatives. De plus, aucune des ces méthodes ne permet un enrichissement de la population de cellules issues du sang maternel en cellules foetales qui soit suffisamment important pour envisager en routine, un diagnostic prénatal, spécifique et sensible. La sensibilité d'un diagnostic sur cellules foetales (et donc sa fiabilité) est en fait directement corrélée à la pureté des préparations de cellules foetales utilisées pour le diagnostic. Or, aucune des méthodes d'enrichissement citées précédemment ne permettent l'obtention de populations de cellules foetales non apoptotiques et suffisamment pures, en particulier non exemptes de cellules d'origine maternelle.

Il existe par conséquent, un réel besoin de développer de nouvelles méthodes de diagnostic non-invasives dont la sensibilité et la spécificité du diagnostic seraient très élevées, et de manière préférée, proches de 100%.

Au vu de l'état de la technique et de la nature des cellules foetales circulantes dans le sang maternel, l'invention résulte de la constatation que seul un procédé permettant une concentration suffisante en cellules foetales, associé à leur examen morphologique, et à l'analyse génétique ciblée sur le génome de cellules uniques, est susceptible de surmonter les inconvénients des méthodes existantes.

Un procédé de séparation des cellules épithéliales pathogéniques circulantes dans le sang, et en particulier des cellules tumorales par filtration d'un échantillon de liquide biologique, est décrit dans la demande de brevet FR 2 782 730. Celui-ci est basé sur la constatation que la taille des cellules épithéliales circulantes est supérieure à celle des cellules leucocytaires. Vona *et al.* (9) ont utilisé ce procédé de filtration pour détecter et identifier différentes lignées cellulaires cancéreuses mélangées en très faible concentration dans un échantillon de sang (1 à 3 cellules par millilitre de sang) ainsi que des cellules tumorales isolées de patients atteints de cancer du foie.

Les inventeurs ont maintenant montré qu'il est possible de combiner la méthode de filtration des cellules décrites par Vona *et al.,* l'analyse immunologique ou cytologique des cellules retenues par filtration et une méthode d'analyse génétique ciblée sur des cellules individuelles pour obtenir un enrichissement suffisant des cellules d'origine foetale et la mise en oeuvre d'un diagnostic prénatal sur le génome de cellules foetales individuelles isolées du sang maternel et dont l'origine foetale est clairement démontrée. En particulier, la présente invention permet d'enrichir une population de cellules issues du sang maternel en cellules foetales épithéliales d'un facteur supérieur à six millions. Il est en effet possible de détecter par la méthode de l'invention, de 1 à 7 cellules foetales pour 2 ml de sang maternel analysé. Compte tenu de ces données, on peut donc en conclure que le procédé de l'invention permet d'isoler la quasi-totalité des cellules foetales présentes dans le sang maternel. En outre, après filtration et collecte des cellules par microdissection, les inventeurs ont montré qu'il est possible d'observer deux types cellulaires caractéristiques, l'un mononucléé de type cytotrophoblaste d'un diamètre compris entre 14 et 20 µm environ et l'autre plurinucléé de type syncytiotrophoblaste d'un diamètre d'environ 44-47 µm ou plus.

Une étude menée sur 13 femmes à 11-12 semaines de grossesse, a démontré qu'il est possible de diagnostiquer le sexe masculin des foetus par l'analyse de 2 millilitres de sang maternel *via* la détection de séquences spécifiques du chromosome Y dans le génome de cellules uniques microdisséquées. Il a en outre été montré qu'il est possible de déterminer par allèlotypage, l'origine foetale ou maternelle de ces mêmes cellules isolées.

Ainsi, la présente invention découle, en partie, de la découverte que les cellules foetales peuvent être concentrées par la mise en oeuvre du procédé de filtration tel que décrit dans la demande FR 2 782 730. L'invention découle aussi en partie de la démonstration que l'analyse morphologique des cellules retenues sur le filtre permet d'établir une présomption sur l'origine foetale ou maternelle de ces cellules et leur analyse génétique permet la démonstration de leur origine foetale ou maternelle. En particulier, elle démontre qu'un diagnostic prénatal peut être réalisé sur un génome foetal pur, obtenu par microdissection de cellules uniques. Enfin, l'invention découle de la démonstration que la méthode de diagnostic prénatal peut être mise en oeuvre à une période précoce de la grossesse et sur un volume limité de sang maternel prélevé.

Par « Diagnostic prénatal », il faut comprendre à la fois la recherche d'une caractéristique particulière du foetus (par exemple le sexe) ou encore la recherche d'une anomalie génétique ou de tout type de pathologie génétique (altération de l'ADN), infectieuse (virale, bactérienne ou parasitaire) ou métabolique (altération de la synthèse des ARN messagers et/ou des protéines) pouvant être détecté à partir de l'analyse génétique des cellules foetales isolées.

Ainsi, selon les modes de réalisation de l'invention choisis, le diagnostic prénatal consiste à rechercher une anomalie génétique ou chromosomique sur l'ADN d'une cellule foetale, une pathologie génétique ou infectieuse (virale, bactérienne ou parasitaire), ou, un génotype précis, et en particulier le sexe génétique du foetus.

Par « Méthode peu invasive ou non invasive» , il faut comprendre une méthode qui n'implique pas de prélèvement de tissus ou cellules foetales par biopsie et/ou effraction de la barrière placentaire.

Ainsi, l'invention porte sur une méthode de diagnostic prénatal sur cellules foetales isolées du sang maternel comprenant les étapes suivantes :
a) la filtration d'un échantillon de sang maternel pur ou dilué, de façon à concentrer sur un filtre selon leur taille certaines cellules circulantes et en particulier des cellules d'origine foetale;
b) l'analyse des cellules retenues sur le filtre afin d'obtenir une présomption ou une identification de leur origine foetale ou maternelle ; et l'identification de leur nature épithéliale,
c) la démonstration de l'origine foetale de certaines cellules enrichies par analyse génétique de cellules isolées individuellement,
d) la recherche d'anomalies génétiques ciblée spécifiquement aux génomes cellulaires analysés individuellement et dont l'origine foetale a été démontrée.

Le diagnostic prénatal, pour être fiable, doit être réalisé sur un génome foetal pur. Le terme « présomption » ou « présumée » à l'étape (b) indique donc la forte probabilité d'être en présence d'une cellule d'origine foetale.

Lors de l'étape (c), l'analyse est réalisée de préférence par analyse génétique à l'aide de marqueurs spécifiques des génomes foetaux, comme cela est expliqué en détail plus loin.

La ou les caractéristique(s) des cellules foetales susceptibles d'être recherchée(s) dans l'étape (d) peuvent être soit une anomalie génétique ou chromosomique du foetus, soit un génotype particulier de ce dernier.

La recherche de cette caractéristique particulière est, de manière préférée, réalisée par analyse génétique sur cellules uniques dont l'origine foetale est préalablement démontrée.

Pour les besoins du diagnostic prénatal selon l'invention, un échantillon de sang est prélevé de la femme enceinte. Dans un mode de réalisation particulier de l'invention, un échantillon de sang maternel est prélevé précocement au cours de la grossesse (par exemple autour de la cinquième semaine de grossesse). Cependant, le prélèvement de l'échantillon de sang maternel et la méthode de diagnostic selon l'invention peuvent être réalisés à tout moment, du début jusqu'à la fin de la grossesse. Dans un mode de réalisation préféré de l'invention, le prélèvement et la méthode de diagnostic sont réalisés entre la 7^{ème} et la 15^{ème} semaine de grossesse. Dans un autre mode de réalisation, le prélèvement et la méthode de diagnostic sont réalisés entre la 10^{ème} et la 15^{ème} semaine de grossesse. On prélèvera généralement entre 3 et 20 millilitres de sang maternel, de préférence entre 5 et 10 millilitres. Si, pour des raisons pratiques, on prélève entre 3 et 20 millilitres de sang maternel, il faut bien comprendre que l'analyse ultérieure des cellules foetales isolées décrite ci-après, peut être réalisée sur un volume d'échantillon de sang prélevé plus limité, par exemple compris entre 1 et 10 millilitres, de préférence entre 2 et 5 millilitres. Afin d'augmenter la sensibilité du diagnostic, il est possible de réaliser plusieurs prélèvements indépendants afin de répéter le diagnostic sur différents échantillons indépendants. En outre, dans un mode de réalisation préféré de l'invention, il est possible de prélever en parallèle un échantillon de sang chez le père; et un échantillon de cellules chez la mère, par exemple par prélèvement de cellules buccales par « scraping » ou de sang avant la grossesse, et de réaliser à partir du matériel prélevé, une recherche de marqueurs spécifiques du génome paternel et du génome maternel. Cette étude parallèle permet d'identifier des marqueurs génétiques spécifiques du père et de la mère, qui pourront être utilisés pour démontrer l'origine foetale des cellules isolées par filtration selon le mode de réalisation décrit plus loin.

L'invention résulte notamment de l'observation que les cellules foetales épithéliales circulantes dans le sang ont un diamètre supérieur aux cellules leucocytaires et érythrocytaires maternelles et peuvent être isolées selon des procédés de filtration adaptés de ceux décrits pour l'isolement de cellules pathogéniques circulantes dans le sang tels que ceux décrits dans la demande de brevet FR 2782730.

Ainsi, selon la méthode de l'invention, l'échantillon de sang prélevé est filtré de façon à concentrer sur le filtre, selon leur taille, certaines cellules circulantes d'origine foetale ou maternelle et les séparer ainsi des cellules sanguines, en particulier des leucocytes maternels.

Préalablement à l'étape (a) de filtration, tout procédé permettant l'enrichissement de la population cellulaire issue de l'échantillon de sang, en cellules d'origine foetale peut être mis en oeuvre. Dans une forme de réalisation de l'invention, un enrichissement de la population en cellules foetales est réalisé par le tri des cellules en fonction de l'expression de marqueurs de surfaces exprimés par les cellules foetales pour diminuer la proportion de cellules d'origine maternelle. Les techniques de tri des cellules sont par exemple le FACS, le tri sur colonne d'immunoaffinité ou immunomagnétique (MACS) ou toute technique susceptible d'obtenir un enrichissement d'un type cellulaire sur la base de caractéristiques physiques (densité) ou structurelles (antigènes spécifiques notamment).

Afin de faciliter la filtration, dans un mode de mise en oeuvre particulier, on procède avant l'étape de filtration à la dilution de l'échantillon de sang dans une solution de filtration, ladite solution de filtration consistant en un réactif de fixation des cellules nucléées et/ou de lyse des globules rouges. Un exemple de solution de filtration comprend un détergent capable de dégrader la membrane des globules rouges, tel que la saponine, et un fixateur capable de stabiliser la membrane des cellules nucléées tel que le formaldéhyde.

Dans un mode de mise en oeuvre préféré, l'échantillon de sang maternel est dilué de 10 à 100 fois environ dans la solution de filtration.

La filtration de l'échantillon de sang maternel pur ou dilué est réalisée à l'aide d'un filtre poreux permettant de séparer les cellules selon leur taille. La porosité du filtre est choisie de manière à laisser passer les éléments figurés du sang, notamment les érythrocytes, les plaquettes et les leucocytes maternels et à retenir certaines cellules nucléées et en particulier des cellules larges (épithéliales ou précurseurs hématopoïétiques) d'origine maternelle et foetale.

Avantageusement, on utilise un filtre de porosité comprise entre 6 et 15 µm et d'une densité adaptée selon la porosité choisie et apte à retenir les cellules tout en évitant le colmatage de celles-ci lors de la filtration. De préférence, le filtre présente des pores sensiblement cylindriques d'un diamètre d'environ 8 µm et de densité comprise entre 5.10⁴ et 5.10⁵ pores/cm². De manière encore préférée, le filtre utilisé est calibré de façon à ce que l'ensemble des pores présente un diamètre sensiblement identique. Un exemple de filtre utilisable dans le procédé de l'invention est une membrane filtrante calibrée de polycarbonate de type « Track-Etched membrane » d'une densité de pores de 1x10⁵ pores/cm², d'une épaisseur de 12µm et d'une taille des pores de 8µm, telle que celle commercialisée par Whatman®.

Les cellules retenues sur le filtre peuvent être observées sous microscope, par exemple après coloration à l'hématoxyline et l'éosine de manière à analyser leur morphologie. Leur nature épithéliale peut être identifiée, à titre d'exemple, par immunomarquage avec un anticorps anti-cytokeratines (type KL₁). Il est à ce stade possible d'envisager de reconnaître sur la base de caractères morphologiques les cellules d'origine foetale et en particulier, les cellules cytotrophoblastiques, mononucléées, présentant un grand noyau, une chromatine condensée et un cytoplasme réduit, d'un diamètre compris entre 14 et 20 µm et les cellules syncytiotrophoblastiques d'un plus grand diamètre (44-47 µm ou plus) et plurinucléées.

L'analyse génétique des cellules retenues sur le filtre permet d'obtenir des indications sur l'origine foetale ou maternelle de chacune de ces cellules. En particulier, en vue d'un diagnostic sensible et spécifique, l'étape de filtration sera répétée si aucune cellule présumée d'origine foetale n'est observée sur le filtre après une première filtration. Selon les modes de réalisation, l'analyse génétique sera établie sur l'ensemble des cellules retenues sur le filtre et comprenant certaines cellules dont l'origine foetale est présumée, ou sur le génome de cellules isolées individuellement.

Dans un mode de mise en oeuvre particulier, on procède à l'analyse de présomption de l'origine foetale ou maternelle des cellules retenues sur le filtre par la recherche de la présence de marqueur(s) immunologique(s) ou cytologique(s) caractéristique(s) des cellules foetales.

On entend par marqueur immunologique caractéristique des cellules foetales tout antigène ou combinaison d'antigènes dont l'expression est significativement différente entre les cellules foetales et les cellules maternelles, et qui peut être détectée à l'aide d'un anticorps ou d'une combinaison d'anticorps dirigés spécifiquement contre ledit antigène ou combinaison d'antigènes. Des exemples de tels marqueurs immunologiques sont en particulier les antigènes associés aux cellules trophoblastiques décrits dans la demande de brevet WO 90/06509. La recherche de la présence de marqueurs immunologiques caractéristiques des cellules foetales consiste par exemple :
- en la mise en contact des cellules contenues dans l'échantillon de sang maternel avec au moins un anticorps dirigé contre un antigène caractéristique des cellules foetales ; et,
- la détermination sur les cellules retenues sur le filtre d'une liaison spécifique dudit anticorps avec un antigène exprimé à la surface desdites cellules,
ladite mise en contact des cellules avec l'anticorps pouvant être réalisée avant ou après l'étape de filtration. Les anticorps choisis peuvent être du type polyclonal ou monoclonal.

Un exemple d'antigène caractéristique des cellules foetales est l'antigène de la phosphatase alcaline placentaire.

Dans un autre mode de mise en oeuvre, on peut procéder à l'analyse de présomption de l'origine foetale des cellules par la détermination de marqueurs cytologiques spécifiques des cellules cytotrophoblastiques et/ou syncyciotrophoblastiques. Les marqueurs cytologiques utilisables comprennent l'ensemble des caractéristiques cytologiques des cellules foetales permettant de les différencier des autres types de cellules circulantes susceptibles d'être retenues sur le filtre, en particulier, la taille des cellules, la forme des cellules, la présence et la taille d'organites particuliers, la taille et le nombre de noyau, la structure de la chromatine, etc.., ou toutes combinaisons particulières de ces caractéristiques cytologiques. Les caractères cytologiques peuvent être observés par coloration des cellules à l'aide de colorants classiquement utilisés en cytologie, en particulier l'hématoxyline-éosine et par l'observation des cellules marquées en microscopie optique.

Pour la recherche d'une anomalie chromosomique ou du sexe du foetus, dans un mode de mise en oeuvre particulier, on procède à l'hybridation *in situ* de sondes spécifiques de l'anomalie chromosomique ou du sexe à détecter, sur le génome des cellules retenues sur le filtre, et à la recherche d'une hybridation spécifique sur le génome des cellules dont l'origine foetale est présumée. Des sondes spécifiques d'une séquence chromosomique peuvent notamment être des sondes d'ADN ou de type PNA (peptid nucleic acid) (11). Un exemple de technique d'hybridation in situ est connu sous le nom de la méthode FISH (Fluorescence In Situ Hybridization) (10), mais toute méthode connue de l'homme du métier permettant de détecter sur le génome d'une cellule une anomalie chromosomique ou les chromosomes sexuels à l'aide de sondes spécifiques, peut être utilisée dans le cadre de la méthode de l'invention.

Citons à titre d'exemples d'anomalies chromosomiques pouvant être détectées, la trisomie 13, la trisomie 18, la trisomie 21, le syndrome de Turner, le syndrome Penta X, le syndrome XYY ou le syndrome de Klinefelter.

Dans un autre mode de réalisation particulièrement préféré de l'invention, après l'analyse de présomption de l'origine foetale ou maternelle des cellules, les cellules dont l'origine foetale est présumée sont collectées individuellement. Par collecte individuelle des cellules, il faut comprendre toute méthode qui permet de collecter une cellule individuelle spécifique retenue sur le filtre en vue de son analyse ultérieure indépendamment des autres cellules retenues sur le filtre. Dans un mode de réalisation préféré, des cellules retenues sur le filtre sont collectées individuellement par microdissection. La collecte individuelle d'une cellule par microdissection consiste à découper au laser la partie de la membrane filtrante sur laquelle est retenue une cellule, ou à décoller la cellule du filtre à l'aide du laser, puis à récupérer la cellule unique collectée dans un tube approprié. Celle-ci est alors apte à subir les différentes analyses permettant le diagnostic prénatal telles qu'exposées plus loin.

La collecte individuelle des cellules permet de cibler avantageusement l'analyse génétique sur le génome d'une cellule unique. Elle permet en outre de réaliser la recherche d'une anomalie génétique ou chromosomique du foetus ou d'un génotype particulier de celui-ci sur le génome d'une cellule unique dont l'origine foetale a été préalablement démontrée par analyse génétique. Ainsi, par ce mode de réalisation de l'invention, on obtient de manière avantageuse du matériel génétique pur, c'est à dire, dérivé d'une cellule unique, et utilisable à la fois pour la démonstration de l'origine foetale de la cellule analysée puis pour la recherche éventuelle d'une anomalie génétique ou d'une caractéristique de celle-ci, ou d'un génotype particulier.

Tout type de méthode d'analyse génétique peut être utilisé pour démontrer l'origine foetale ou maternelle d'une cellule unique collectée ou procéder à la recherche d'anomalies génétiques du foetus ou d'un génotype particulier de celui-ci dès lors que ces méthodes sont suffisamment sensibles pour identifier sur une cellule unique le ou les caractères recherchés.

Une méthode d'analyse préférée pour la démonstration de l'origine foetale est l'analyse par allèlotypage de l'ADN des cellules collectées, en particulier par l'identification de marqueurs microsatellites spécifiques de l'ADN paternel et maternel.

Il est par conséquent possible de réaliser
- la démonstration de l'origine foetale ou maternelle des cellules, puis ,
- le diagnostic prénatal par la recherche d'anomalies génétiques ou chromosomiques du foetus ou d'un génotype particulier de celui-ci,
par l'identification sur une préparation d'ADN dérivée d'une cellule unique collectée, d'un ou plusieurs marqueur(s)/cible(s) génétique(s) ou de polymorphisme ou d'une combinaison de ces marqueurs/cibles, ou d'un dosage allélique particulier de ces marqueurs ou d'une cible génétique.

Dans le texte qui suit, on préférera le terme « cible génétique » pour faire référence à la caractéristique génétique recherchée, y compris le polymorphisme, dans le cadre du diagnostic prénatal et le terme « marqueur » pour faire référence aux éléments permettant la démonstration de l'origine foetale ou maternelle des cellules analysées.

On entend par cible génétique, toute caractéristique génétique, par exemple une mutation particulière d'un gène, associée spécifiquement à un phénotype ou à une pathologie génétique ou infectieuse du foetus.

Par marqueur de polymorphisme, il faut comprendre toute caractéristique identifiable sur l'ADN dont la présence est corrélée à un génotype particulier. Ces marqueurs permettent de distinguer l'ADN paternel de l'ADN maternel et donc de démontrer la composition bi-patentale de l'ADN foetal. On peut citer par exemple les marqueurs de polymorphisme de longueur de fragments de restriction (RFLP), les marqueurs SNP (Single Nucleotide Polymorphism), les marqueurs micro-satellites, les marqueurs VNTR (Variable Number of Tandem Repeats) ou STR (Short Tandem Repeats).

Les marqueurs micro-satellites sont particulièrement préférés pour la caractérisation des cellules et la mise en oeuvre du diagnostic prénatal. Dans un mode de réalisation de l'invention, au moins un marqueur de polymorphisme à identifier est un marqueur microsatellite, un marqueur VNTR (Variable Number of Tandem Repeats) ou un marqueur STR (Short Tandem Repeats). Ceux-ci présentent en effet l'avantage d'être identifiables par l'amplification à l'aide d'amorces spécifiques. Les marqueurs micro-satellites, VNTR ou STR, sont composés de répétitions en tandem, le plus souvent de motifs polyCA/GT. Les variations alléliques, dues à la variation du nombre des répétitions, sont aisément détectées par amplification de type PCR, grâce à l'utilisation d'amorces correspondant aux séquences uniques flanquant le microsatellite. Une carte physique de ces marqueurs microsatellites et la séquence de leurs amorces associées sont décrits par Dib *et al.* (12). Par cette méthodologie, la mise en évidence d'une contribution biparentale au génotype des cellules analysées permet d'établir de manière certaine l'origine foetale des cellules analysées. En outre, la présence de marqueurs microsatellites particuliers peut être spécifiquement recherchée, notamment en tant que cible génétique, pour le diagnostic prénatal, notamment pour le diagnostic d'altérations chromosomiques particulières.

Afin de démontrer l'origine foetale ou maternelle d'une cellule unique collectée, dans un mode de mise en oeuvre particulier, on procède à la recherche d'un marqueur ou d'une combinaison de marqueurs ou d'un dosage allélique de ces marqueurs se distinguant de ceux du génome de cellules maternelles, en particulier par la recherche sur le génome de ladite cellule collectée, d'un marqueur ou d'une combinaison de marqueurs spécifique de l'ADN de cellules paternelles. Leur présence est nécessairement la signature d'une origine foetale de la cellule en cause.

Dans le cas où le sexe du foetus est connu et de sexe masculin, un marqueur spécifique de l'ADN des cellules foetales peut être un marqueur génétique spécifique du chromosome Y ou une combinaison de tels marqueurs.

Avantageusement, préalablement à la démonstration de l'origine foetale ou maternelle d'une cellule collectée et/ou à la recherche d'une anomalie génétique ou chromosomique du foetus ou d'un génotype particulier de celui-ci, ladite cellule collectée est lysée et l'ensemble de son génome est pré-amplifié, par exemple à l'aide d'amorces génériques couvrant toutes les séquences possibles selon les méthodes connues de préamplification par extension d'amorces (PEP, Primer Extension Preamplification) (13) ou encore la méthode DOP-PCR. Ces méthodes permettent d'amplifier la totalité du génome d'une seule cellule. La préparation d'ADN pré-amplifié ainsi obtenue et dérivée de l'ADN d'une cellule unique peut alors être purifiée et utilisée en tant que matériel génétique pour la détection spécifique de marqueurs génétiques ou de polymorphisme et/ou la détection d'une cible génétique.

De manière préférée, on procède à la démonstration de l'origine foetale ou maternelle d'une cellule collectée par l'amplification de marqueurs génétiques ou de polymorphisme ou d'une combinaison de ces marqueurs, à partir de la préparation d'ADN pré-amplifié dérivée de l'ADN d'une cellule unique. Les marqueurs génétiques capables de mettre en évidence la contribution bi-parentale à l'ADN foetal sont identifiés par l'analyse préalable de l'ADN paternel et maternel. On peut procéder à la recherche d'une cible génétique en vue du diagnostic prénatal à partir de la même préparation d'ADN pré-amplifiée dérivée de l'ADN d'une cellule unique par l'amplification d'une ou plusieurs séquence(s) portant la ou les cible(s) génétique(s) recherchée(s). Toute technique permettant l'amplification spécifique d'un acide nucléique donné peut être utilisée dans le procédé de l'invention. Citons à titre d'exemple la méthode d'amplification PCR (Polymerase Chain Reaction) ou les méthodes d'amplification isotherme telles que la TMA (transcription mediated amplification), la NASBA (nucleic acid sequence based amplification), la 3SR (self sustained sequence replication) ou l'amplification par déplacement de brin (strand displacement amplification).

Les méthodes d'amplification et en particulier, la méthode PCR, sont suffisamment sensibles pour être mises en oeuvre à partir de moins d'un cinquième de la préparation d'ADN pré-amplifié. Par conséquent, chaque préparation d'ADN pré-amplifié d'une cellule collectée peut être utilisée pour l'amplification de cinq marqueurs ou cibles génétiques différents au moins.

En particulier, les amplifications réalisées permettent la détection de marqueurs microsatellites. A titre d'illustration, il est présenté, en exemple, la détection de marqueurs microsatellites pour la démonstration de l'origine foetale de cellules isolées selon le procédé de l'invention par amplification PCR des préparations d'ADN préamplifié. L'amplification PCR permet aussi de détecter des cibles génétiques, et notamment des mutations ponctuelles ou de type délétions, ou micro-délétions associées à un caractère génétique ou à une pathologie spécifique. Les séquences susceptibles de porter la délétion sont amplifiées et les produits d'amplification sont séparés selon leur taille, par exemple par électrophorèse. La présence de délétions est détectée par la présence d'un produit d'amplification de taille inférieure aux produits d'amplification ne portant pas de délétion.

Les produits d'amplifications peuvent aussi être séquencés, en particulier pour caractériser de manière précise les marqueurs ou les cibles génétiques recherchés et notamment les mutations ou génotypes recherchés en vue d'un diagnostic prénatal. Ainsi, dans un mode de réalisation particulier, on procède à la démonstration de l'origine foetale ou maternelle d'une cellule collectée et/ou au diagnostic prénatal, notamment à la recherche d'une anomalie génétique ou chromosomique du foetus ou d'un génotype particulier de celui-ci par le séquençage des marqueurs ou cibles génétiques amplifiés. A titre d'exemple, il est possible de détecter par la méthode de l'invention, certaines mutations du gène CFTR associées au diagnostic de la mucoviscidose telle que la microdélétion ΔF 508 du gène CFTR.

Il est encore possible de diagnostiquer la trisomie 21 par l'amplification PCR de marqueurs VNTR du chromosome 21 tels que les marqueurs D21S1414 et D21S1411 et le dosage allélique de ces marqueurs.

Dans un autre mode de mise en oeuvre de la méthode de l'invention, on procède à la démonstration de l'origine fatale ou maternelle d'une cellule collectée individuellement puis au diagnostic prénatal, notamment à la recherche d'une anomalie génétique ou chromosomique du foetus ou d'un génotype particulier de celui-ci par l'hybridation de tout ou partie de la préparation d'ADN pré-amplifié avec des sondes d'ADN spécifiques. Les sondes d'ADN sont choisies de manière à s'hybrider spécifiquement sur les cibles génétiques ou de polymorphismes pour leur identification ou sur les séquences portant les cible(s) génétique(s) à rechercher. L'hybridation des sondes sur les cibles génétiques peut être détectée selon les techniques classiques de détection des complexes d'hybridation d'acides nucléiques de type slot blot, Southern blot ou avantageusement aujourd'hui par des puces à ADN ou micro-réseaux ou macro-réseaux (14). Des sondes moléculaires peuvent par exemple être choisies pour la détection spécifique de la fibrose cystique, des dystrophies musculaires, de la maladie de Gaucher, des hémoglobinopathies, de l'hémophilie, des phénylcétonuries ou de la mucoviscidose.

Ainsi, dans un mode de réalisation de l'invention, les sondes d'ADN spécifiques des cibles génétiques à identifier sont fixées sur un support formant une puce à ADN ou micro-réseaux ou macro-réseaux. La préparation d'ADN pré-amplifié est par exemple marquée à l'aide d'un marqueur radioactif ou fluorescent, et mise en contact avec la puce à ADN ou micro-réseaux ou macro-réseaux comportant les sondes spécifiques. L'intensité d'hybridation est mesurée pour chaque spot contenant une sonde spécifique, déterminant ainsi avec une grande sensibilité la présence des marqueurs recherchés sur l'ADN d'une cellule collectée.

Le choix des des cibles génétiques dépend bien sûr du génotype à rechercher. Des exemples de cibles génétiques utilisables selon les diagnostics prénatals sont décrits dans l'état de la technique.

Une méthode alternative pour déterminer des anomalies chromosomiques et notamment des gains et pertes de chromosomes en vue d'un diagnostic prénatal est la méthode d'hybridation génomique comparative (CGH), consistant (i) à comparer l'hybridation sur une préparation chromosomique, cosmidique ou sur une puce à ADN, d'une préparation d'ADN pré-amplifié dérivée du génome d'une cellule unique collectée après filtration selon le procédé, et d'une préparation d'ADN pré-amplifié issue de cellules d'origine maternelle ou de cellules de référence non foetales, les deux préparations ayant été marquées à l'aide de marqueurs différents et (ii) à identifier les différences d'hybridation entre l'ADN de la cellule collectée après filtration et l'ADN maternel (16). Ainsi, dans un mode de réalisation de l'invention, on procède au diagnostic prénatal par une méthode d'hybridation génomique comparative (CGH) d'une préparation d'ADN pré-amplifié issue de l'ADN d'une cellule unique collectée et dont l'origine foetale est démontrée, et d'une préparation d'ADN pré-amplifié de cellules d'origine maternelle ou de cellules de référence non foetales.

Un autre aspect de l'invention concerne une méthode d'obtention d'une population de cellules foetales dérivées d'une population de cellules isolée du sang maternel et concentrée en cellules d'origine foetale, ledit procédé comprenant les étapes suivantes :
a) La filtration d'un échantillon de sang pur ou dilué, prélevé d'une femme enceinte, de façon à concentrer sur un filtre selon leur taille certaines cellules circulantes et en particulier les cellules d'origine foetale; et
b) la remise en culture des cellules retenues sur le filtre pour l'obtention d'une culture de cellules concentrées en cellules foetales épithelials.

Le procédé décrit ci-dessus résulte de la constatation par les inventeurs que, de façon inattendue, le procédé de l'invention permet de concentrer de manière particulièrement importante, une population de cellules isolées du sang maternel en cellules d'origine foetale. Elle résulte en outre de la constatation que les cellules peuvent être filtrées même sans fixation préalable, c'est-à-dire avec viabilité conservée.

En outre, à partir des populations de cellules obtenues par le procédé ci-dessus, il est possible d'obtenir des cultures pures de cellules foetales selon les techniques de clonage et d'expansion connues de l'homme du métier. Les populations pures ou enrichies en cellules foetales obtenues par le procédé trouvent leurs applications en particulier dans la préparation d'un produit de thérapie cellulaire comprenant lesdites cellules foetales ou des cellules issues de leur différenciation.

L'ensemble des méthodes décrites ci-dessus reposent sur l'existence et la mise au point d'un dispositif spécifique nommé ISET (acronyme anglais pour Isolation by Size of Epithelial Tumor Cells) et décrit dans EP 0513 139 et comprenant sur un bâti :
- un filtre poreux apte à retenir certaines cellules circulantes selon leur taille, monté entre deux dispositifs de serrage, respectivement en amont et en aval du sens de filtration, et destiné à l'étanchéité de la filtration,
- le bloc amont comprenant des moyens de stockage et/ou de pré-traitement des échantillons à analyser,
- le bloc aval comprenant des perforations en regard des moyens de stockage pour collecter les déchets,
- des moyens de filtration forcée.

Ainsi, l'invention porte sur l'adaptation et l'utilisation d'un dispositif de ce type à la filtration de cellules foetales présentes dans le sang maternel.

Par adaptation et utilisation, on entend :
- l'incorporation dans le dispositif d'un filtre de porosité, de préférence calibré, apte à retenir les cellules d'un diamètre moyen supérieur à 8 µm, et mieux, supérieur à 10 µm, de préférence supérieur à 15 µm. Un filtre de porosité moyenne de 8 µm a été démontré comme répondant à ces caractéristiques recherchées ;
- l'adaptation de la densité des pores sur le filtre, en fonction de taille des pores afin d'optimiser la capacité de filtration,
- l'adaptation de la pression appliquée sur les moyens de filtration à la conservation de l'intégrité physique des cellules foetales recherchées ;
- l'adaptation du milieu de dilution de l'échantillon de sang maternel à la conservation de l'intégrité et de la viabilité des cellules recherchées.

L'invention porte plus particulièrement sur l'utilisation d'un dispositif de filtration de type ISET, pour l'isolement de cellules foetales du sang maternel et comportant un filtre de porosité moyenne comprise entre 6µm et 15 µm, et de préférence d'environ 8 µm.

Elle porte aussi sur l'utilisation d'un dispositif de type ISET dans lequel le filtre présente des pores d'un diamètre d'environ 8 µm et une densité de pores comprise entre 5.10⁴ et 5.10⁵.

Elle porte enfin sur l'utilisation d'un dispositif de filtration de type ISET dans lequel la pression de filtration appliquée est comprise entre 0,05 bars et 0,8 bars, et de préférence environ 0,1 bars.

Les exemples ci-après et les figures qui suivent illustrent un mode de réalisation de la méthode de diagnostic prénatal et son application à la détection du sexe foetal. Ils en démontrent en outre sa sensibilité et sa spécificité.

Plus précisément, dans l'exemple qui suit, la méthode de diagnostic a été mise en oeuvre sur les cellules isolées du sang de 13 femmes enceintes. La méthode mise en oeuvre comprend la détection des cellules foetales par leur analyse morphologique et des marqueurs spécifiques du sexe masculin (amorces d'amplification des séquences du chromosome Y). Elle comprend en outre la démonstration de l'origine foetale des cellules retenues sur le filtre par allélotypage au moyen de l'amplification PCR de marqueurs microsatellites. Les résultats montrent que la méthode de diagnostic est particulièrement sensible puisque, chez les mères portant des foetus de sexe féminin, 100% des cellules sont négatives au test du chromosome Y. De plus, l'analyse de marqueurs microsatellites spécifiques (STR, ou Short Tandem Repeats) sur 11 cellules isolées révèlent que 6 d'entre elles ont un profil de type « cellules foetales » et 5 ont un profil « maternel ». Ces résultats sont en accord avec les résultats obtenus sur la détection du chromosome Y. Ils constituent la preuve moléculaire que le test permet, en parallèle, la détection spécifique de cellules foetales et la réalisation d'un diagnostic prénatal particulièrement sensible et spécifique.

Bien entendu, cet exemple n'est pas limitatif, et la méthode de l'invention est applicable au diagnostic prénatal de toute caractéristique foetale dès lors que celle-ci peut être identifiée à partir d'une cellule foetale épithéliale isolée, individualisée et caractérisée, selon les moyens décrits ci-dessus.

### LEGENDE DES FIGURES

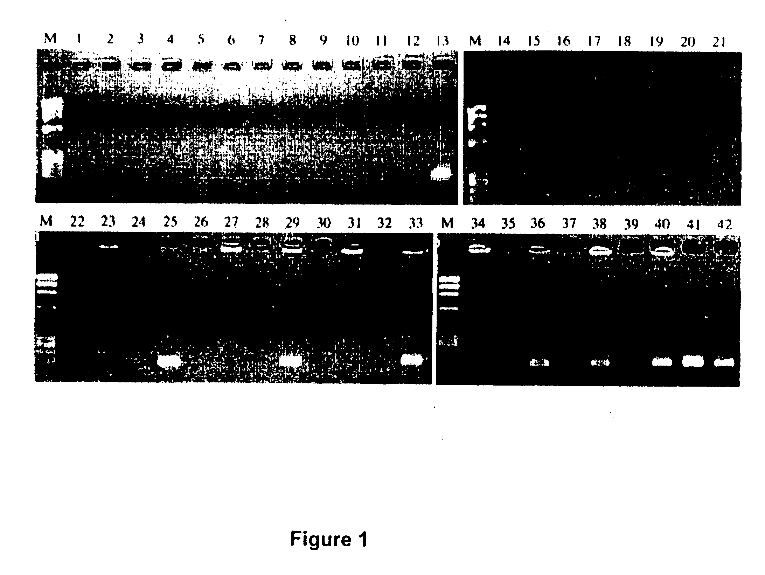

- Figure 1 :: gel de polyacrylamide des produits d'amplification par PCR réalisée sur les cellules trophoblastiques obtenues par le procédé de l'invention. Les différents points numérotés 1 à 42 représentent :
Pistes 1 à 21 : test de spécificité des amorces Y par l'amplification de l'ADN obtenu de 20 femmes (pistes 1 à 12 et 14 à 21) et d'un homme (piste 13, contrôle positif). Le test est négatif pour les échantillons d'ADN de femmes, et positif pour l'ADN d'un homme.
Pistes 22 à 42 : test PCR des amorces Y sur les cellules individuelles isolées du sang de mère portant un foetus de sexe masculin (pistes 23, 25, 27, 29, 31, 33, 34, 36 et 38).
Le test des amorces Y est positif pour l'ADN des cellules foetales de sexe masculin des pistes 25, 29, 33, 36 et 38.
Le test des amorces Y est négatif pour l'ADN des cellules maternelles pistes 23, 27, 31 et 34.
Pistes 22, 24, 26, 28, 30, 32, 35, 37 et 39 : Résultat des contrôles négatifs correspondant au tampon sans échantillon inséré à l'étape de lyse des cellules et conduit jusqu'à la fin du test.
Pistes 41 et 42 : Résultat des contrôles positifs : Une cellule HuH6 (piste 40), 5 ng et 2 ng d'ADN dérivé de leucocytes du sang de 3 hommes (pistes 41 et 42).
M : Marqueur de poids moléculaire (ΦX174 Haelll digéré)
- Figure 2 :: analyse en microscopie des caractéristiques morphologiques des cellules foetales circulantes isolées par ISET.
A : Cellules polynucléées syncitiotrophoblastiques
B : Cellules mononucléées présentant une morphologie de cytotrophoblastes, observée sur un filtre. Deux pores vides sont visibles en haut de la photo.
- Figure 3 :: génotypage à l'aide de marqueurs STR d'ADN parental et trophoblastique et d'ADN de cellules isolées du sang maternel et collectées individuellement après filtration.

Les électrophoretogrammes des produits amplifiés obtenus à l'aide des amorces spécifiques du marqueur STR (Short Tandem Repeat) D1653018 sont présentés.
A : Le marqueur STR utilisé dans ce cas ne permet pas de distinguer l'origine maternelle (M) ou paternelle (F) de l'ADN (état hétérozygote, allèles 250 pb et 262 pb). Cependant, l'ADN trophoblastique (T) présente un état homozygote pour un allèle (250 pb) et un profil identique est retrouvé pour une cellule foetale (FC). Au contraire, une autre cellule microdisséquée (cellule maternelle, MC) présente un profil clairement maternel (état hétérozygote).
B : Dans cet exemple, le marqueur STR permet de distinguer l'origine paternelle ou maternelle des cellules. En effet, l'ADN trophoblastique (T) présente un allèle de 256 bp, aussi retrouvé sur l'ADN maternel (M) ; en revanche, l'ADN paternel présente un allèle différent de 258 bp. Les deux allèles, 256 bp et 258 bp, ont été détectés sur deux cellules microdisséquées, démontrant ainsi leur origine foetale (FC).

### Exemple de mise en oeuvre de la méthode de diagnostic prénatal sur cellules foetales isolées du sang maternel : application à la détermination du sexe du foetus

### Résumé de l'étude

L'étude a porté sur 13 femmes, enceintes de 11 à 12 semaines, portant un foetus présentant un risque de pathologie génétique (6 foetus de sexe masculin et 7 foetus de sexe féminin) et recruté avec leur consentement. Deux millilitres de sang prélevé et récupéré dans un tampon EDTA ont été analysés. Le prélèvement a été réalisé avant tout protocole invasif et l'échantillon de sang a été filtré au plus tard 4 heures après son prélèvement. Le diagnostic du sexe du foetus (par l'analyse de l'ADN) a été établi par prélèvement de villosités choriales.

### A. Méthodes

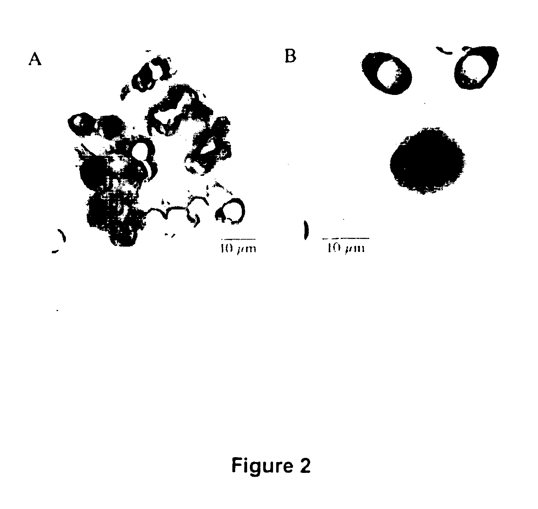

Les échantillons de sang ont été dilués 10 fois dans un tampon de filtration contenant 0,175% de saponine, 0,2% de paraformaldéhyde, 0,0372% d'EDTA et 0,1% de BSA, puis filtrées à l'aide d'un filtre calibré de polycarbonate présentant des pores calibrés cylindriques de 8 µm de diamètre. Les cellules retenues sur le filtre ont été regroupées sur un spot circulaire de 0,6 cm de diamètre. Après coloration à l'éosine et à l'hématoxyline, les spots ont été analysés au microscope et une photographie de chaque cellule a été prise à fort et faible agrandissement. La taille des cellules a été déterminée à l'aide du logiciel Adobe Photoshop, en prenant comme référence la taille de 8 µm des pores. Les photos permettent de retrouver les cellules au microscope Pixcell Il Arcturus (Mountain View, CA). La figure 2 illustre l'analyse microscopique obtenue par cette méthode.

On a réalisé la microdissection de chaque cellule par capture au laser sans aucun traitement préalable du filtre. Afin de s'assurer qu'une seule cellule est collectée à chaque fois, on a pris des photographies du filtre avant et après la microdissection et de la cellule microdisséquée déposée sur la capsule (CapSure^{™} HS). La cellule a ensuite été lysée dans 15 µl de tampon de lyse (100 mM Tris-HCl pH 8, 400 µg/ml proteinase K) pendant 16 heures à 37°C. Le lysat a été collecté après centrifugation et la protéinase K a été inactivée à 90°C pendant 10 minutes. Après une préamplification par extension d'amorces (PEP) comme décrit par Zhang *et al*. (voir plus haut), l'ADN a été précipité à l'éthanol et resuspendu dans 10 µl d'eau. Chaque échantillon a ensuite été testé, d'une part, avec les amorces HLA suivantes :
5'-GTGCTGCAGGTGTAAACTTGTACCAG-3'
5'-CACGGATCCGGTAGCAGCGGTAGAGTT-3',
les amorces HLA permettant de tester l'amplifiabilité de l'ADN (contrôle positif de l'amplification), et d'autre part, avec les amorces STR-spécifiques suivantes :
Marqueur D16S3018
   (sens) 5'-6-FAM-GGATAAACATAGAGCGACAGTTC-3' et,
   (anti-sens) 5'-AGACAGAGTCCCAGGCATT-3';
Marqueur D16S3031
   (sens) 5'-TET-ACTTACCACTGTGCCAGTTG-3' et,
   (antisens) 5'-ATACATGGGTCCTTAAACCG-3' ;
Marqueur D16S539
   (sens) 5'-HEX-GATCCCAAGCTCTTCCTCTT-3' et,
   (antisens) 5'-ACGTTTGTGTGTGCATCTGT-3'.

Les échantillons ont aussi été testés avec les amorces spécifiques du chromosome Y (amorces Y: Y1.7 et Y1.9 telles que décrites dans la référence 17). Les PCR ont été mises en oeuvre sur un volume de mélange réactionnel de 20 µl contenant 2 µl de produit PEP, 10 mM Tris-HCl, 50 mM KCI, 1,5 mM MgCl₂, 0,01% gélatine, 200 mM de chaque désoxynucléotide, 20 picomoles de chaque amorce Y, HLA ou STR et 1 U de Taq polymérase (Perkin-Elmer Cetus, Emeryville, CA). Après une étape de dénaturation initiale à 94°C pendant 5 minutes, on a procédé à 40 cycles d'amplification (94°C 15 s, 60°C 30 s, 72°C 30 s) puis à une étape finale d'élongation à 72°C pendant 5 minutes dans un thermocycleur Perkin Elmer 9700. Des aliquots de 10 µl de produits d'amplification ont été analysés par électrophorèse sur un gel de 2% d'agarose.

Les résultats sont indiqués sur la figure 1. Les conditions de PCR pour l'amplification à l'aide d'amorces spécifiques du chromosome Y ont été établies par l'amplification de 5 ng d'ADN dérivé de leucocytes du sang de 3 individus masculins puis par l'amplification du produit d'ADN pré-amplifié de cellules HuH6 (dérivées d'un carcinome hépato-cellulaire) filtrées et microdisséquées. Pour la PCR à l'aide des amorces STR-spécifiques, après une dénaturation de l'ADN à 94°C pendant 5 minutes, on a procédé à 40 cycles d'amplification (94°C 30", 54°C 30", 72°C 20") suivi d'une phase d'élongation à 72°C pendant 5 minutes. Deux microlitres du premier produit de PCR ont été réamplifiés en utilisant les mêmes conditions PCR. Un microlitre du produit PCR final a été mélangé dans 20 µl de formamide désionisé et 0,4 µl de marqueur Genescan-500 TAMRA puis chargé dans un séquenceur automatique ABI Prism 310. Les profils ont été analysés en utilisant le programme du logiciel Genescan (Perkin Elmer, Foster City, CA). L'allélotypage a été réalisé par amplification, en utilisant les mêmes amorces STR, 1,5 ng d'ADN paternel dérivé de leucocyte du sang et/ou 1,5 ng d'ADN maternel dérivé du sang maternel ou de leucocyte avant la grossesse et 1,5 ng d'ADN trophoblastique obtenu d'un prélèvement de villosités chorioniques.

### Contrôle de la spécificité

La spécificité des amorces Y a été testée par l'amplification de 10 ng d'ADN dérivé de leucocytes du sang de 20 femmes. Les précautions utilisées pour éviter toute contamination des produits de PCR ont été décrites précédemment (18). De plus, un contrôle négatif (le tampon sans l'échantillon) est intercalé pour chaque échantillon à l'étape de lyse et analysé comme les autres échantillons jusqu'à la fin du test. Lors de la réalisation de la microdissection, on a inclus au moins une microdissection d'un nouveau filtre (sans cellules) qui a été menée en parallèle avec les échantillons et les contrôles. On a répété l'amplification avec les amorces spécifiques du chromosome Y ou les amorces STR-spécifiques sur les échantillons et les contrôles, négatifs et positifs, pour vérifier la spécificité des résultats positifs.

### B. Résultats

La spécificité des amorces Y pour l'amplification de l'ADN obtenu de 20 femmes a été déterminée. Le test était négatif pour des échantillons d'ADN de femmes, et positif pour l'ADN d'un homme comme contrôle positif, comme il apparaît dans la figure 1 lorsque l'on compare les lignes 1 à 12, 14 à 21 (femmes) à la ligne 13 (homme).

Un test préliminaire a ensuite été réalisé sur du sang de mère portant un foetus de sexe masculin. Les cellules retenues sur le filtre ont été microdisséquées et analysées à l'aide des amorces spécifiques du chromosome Y et de HLA définies en partie A. Le test avec les amorces HLA était positif pour toutes les cellules et le test avec les amorces Y était positif pour la moitié de ces cellules.

La figure 2 illustre le fait que sur les cellules révélées positives au test, les photographies prises de ces cellules sur le filtre révèlent deux types cellulaires présentant les caractéristiques morphologiques suivantes :
- des cellules mononucléées, de type cytotrophoblaste, d'un diamètre compris entre 14,3 et 19,9 µm (valeur moyenne 16,9 +/- 2), un grand noyau avec de la chromatine condensée et peu de cytoplasme, souvent avec quelques microvillosités à la surface des membranes,
- des cellules polynucléées, de type syncytiotrophoblaste d'un plus grand diamètre (généralement autour de 44-47 µm).

Vingt-trois cellules d'apparence morphologique « foetale » issues d'échantillons de sang de femme portant un foetus de sexe masculin et vingt-six cellules provenant du foetus de sexe féminin ont été ensuite analysées par microdissection et amplification de séquences du chromosome Y. Les résultats sont exprimés dans le tableau 1 ci-après.

**Tableau 1**

| **Mères** | **Cellules collectées** | **Cellules Y positives** | **Cellules mononucléées (Y positives)** | **Cellules polynuclées (Y positives)** |
|---|---|---|---|---|
| Foetus masculin | | | | |
| 1 | 2 | 2 | 1 (1) | 1 (1) |
| 2 | 3 | 2 | 3 (2) | 0 |
| 3 | 8 | 7 | 6 (5) | 2 (2) |
| 4 | 2 | 1 | 2 (1) | 0 |
| 5 | 3 | 1 | 3 (1) | 0 |
| 6 | 5 | 2 | 5 (2) | 0 |
| Total | 23 | 15 | 20 (12) | 3 (3) |
| | | | | |

| **Mères** | **Cellules collectées** | **Cellules Y positives** | **Cellules mononucléées (Y positives)** | **Cellules polynucléées (Y positives)** |
|---|---|---|---|---|
| Foetus féminin | | | | |
| 7 | 6 | 0 | 6 | 0 |
| 8 | 3 | 0 | 1 | 2 |
| 9 | 3 | 0 | 2 | 1 |
| 10 | 2 | 0 | 1 | 1 |
| 11 | 6 | 0 | 5 | 1 |
| 12 | 3 | 0 | 3 | 0 |
| 13 | 3 | 0 | 2 | 1 |
| Total | 26 | 0 | 20 | 6 |

| | | | | |
|---|---|---|---|---|
| Le nombre de cellules Y positives est indiqué entre parenthèses | | | | |

Ce tableau démontre que le test avec les amorces HLA a été positif sur toutes les cellules et le test avec les amorces Y a été positif sur 15 cellules.

Afin de tester la spécificité de ces résultats, les produits d'amplification Y ont été réamplifiés avec les mêmes amorces Y. Tous les contrôles et les cellules négatives à la PCR ont donné un résultat négatif au test, tandis que les cellules positives ont donné un résultat positif, et une cellule faiblement positive (piste 34 de la figure 1) a donné une bande plus forte.

Globalement, la détermination de cellules d'origine foetale a été prouvée pour un nombre variable de cellules (de 1 à 7 cellules Y positives), isolées de 2 ml de sang. Cependant, dans cette étude, l'ensemble des cellules d'apparence morphologique « foetale » n'a pas été microdisséqué. En se basant sur les données morphologiques, une première rapide estimation porte leur nombre à environ 5 cellules par millilitre de sang.

La deuxième partie du tableau indique les résultats d'analyse des cellules foetales de sexe féminin. On observe que le test à l'aide des amorces HLA est positif sur les 26 cellules analysées provenant d'un foetus de sexe féminin, alors que le test à l'aide des amorces spécifiques du chromosome Y est négatif pour l'ensemble des cellules provenant d'un foetus de sexe féminin. Ces résultats démontrent que cette approche permet de détecter spécifiquement des cellule foetales, sans l'existence de faux positifs, et peut être notamment appropriée pour identifier le sexe.

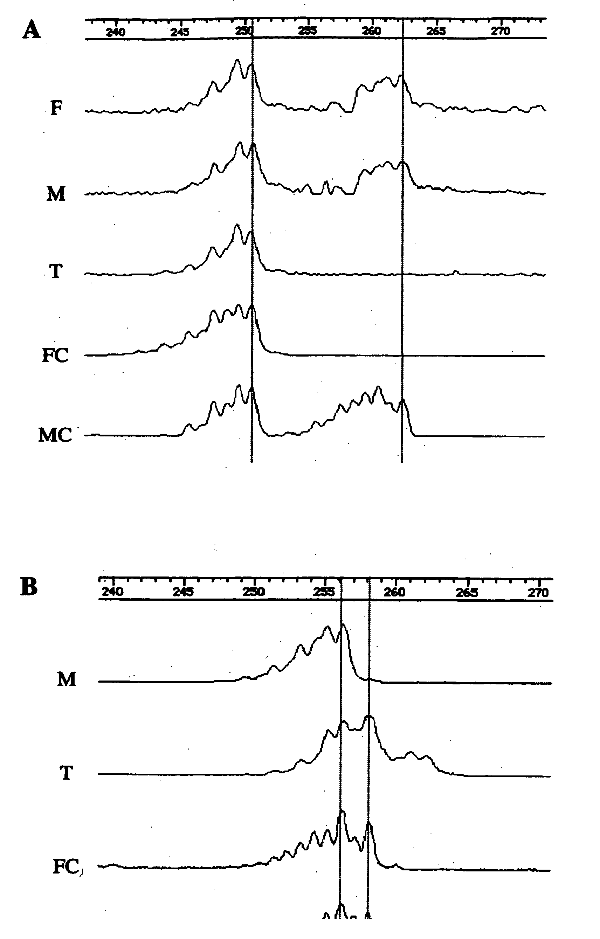

Pour quatre cas, l'ADN de sang paternel et/ou l'ADN de sang maternel (obtenu avant la grossesse) et l'ADN trophoblastique étaient disponibles. Ces échantillons d'ADN ont été utilisés pour identifier l'origine foetale des cellules isolées de manière indépendante de l'identification du sexe. En utilisant trois marqueurs STR et un cinquième de la préparation d'ADN pré-amplifié, il a été ainsi possible de déterminer l'origine foetale ou maternelle de 11 cellules microdisséquées isolées de mère portant un foetus de sexe masculin et aussi testées à l'aide des amorces spécifiques du chromosome Y. Les résultats sont résumés dans le tableau 2 et la figure 3.

**Tableau 2 :**

| **Mère n°*** | **Cellule unique collectée** | **PCR Y spécifique** | **Génotypage sur cellule unique collectée** | | |
|---|---|---|---|---|---|
| | | | **Marqueur D16S539** | **Marqueur D16S3018** | **Marqueur D16S3031** |
| 2 | 2a | + | FC | | |
| | 2b | + | FC | | |
| 3 | 3a | + | | FC | |
| | 3b | + | | FC | |
| | 3c | - | | | MC |
| | 3d | + | | | FC |
| 4 | 4a | - | | MC | |
| | 4b | + | | FC | |
| 6 | 6a | - | | | MC |
| | 6b | - | | | MC |
| | 6c | - | | | MC |

| | | | | | |
|---|---|---|---|---|---|
| * : voir tableau 1 ; MC : cellule maternelle ; FC : profil cellule foetale | | | | | |

### Génotypage STR de cellules uniques isolées du sang maternel et collectées individuellement après filtration

Six cellules présentent un profil foetal en accord avec la détection positive du chromosome Y, et 5 cellules présentent un profil maternel, en accord avec la détection négative du chromosome Y.

Dans un cas (figure 3A), les marqueurs STR testés ne permettent pas de distinguer les allèles paternels (F) des allèles maternels (M) (hétérozygote, 250 et 262 pb), cependant l'ADN trophoblastique (T) présente un état homozygote pour l'un des allèles (250 pb). Le même profil (homozygote pour l'allèle 250 pb) a été détecté sur une cellule foetale (FC) isolée selon le procédé de l'invention, laquelle cellule était positive au test de détection du chromosome Y. Au contraire, une autre cellule microdisséquée, négative au test de détection du chromosome Y présente un profil clairement hétérozygote (cellule maternelle, MC).

Dans un autre cas (figure 3B), l'ADN trophoblastique (T) présente en plus de l'allèle maternel (256 pb) aussi détecté à partir de l'ADN maternel (M), un allèle paternel (258 pb). Ces deux allèles ont été trouvés sur les deux cellules microdisséquées (FC), déterminant ainsi leur origine foetale.

### Discussion

Cette étude montre pour la première fois qu'il est possible de concentrer par filtration, les cellules foetales circulantes dans le sang maternel. Elle montre de plus que l'identification des cellules foetales, indépendamment de l'identification du sexe génétique, peut être réalisée, par l'amplification PCR de marqueurs STR hautement polymorphes. L'impact clinique de la méthode de détection de marqueurs STR dépend en fait de la possibilité de combiner cette méthode avec une approche particulièrement efficace d'enrichissement en cellules foetales. Cette étude montre pour la première fois qu'il est possible de combiner avec succès les deux approches. La technique de séparation par la taille a déjà été décrite pour la séparation de cellules tumorales circulantes dans le sang (Vona *et al.,* voir plus haut). Les inventeurs ont maintenant montré qu'il était possible de concentrer et d'analyser génétiquement les cellules foetales collectées après leur concentration, à partir de seulement deux millilitres d'un échantillon de sang maternel. En combinaison avec la micro-dissection laser et la PCR sur cellule individuelle en utilisant des amorces spécifiques du chromosome Y ou des amorces STR, cette approche a permis la démonstration de l'origine foetale des cellules collectées. En prenant en compte le nombre de cellules foetales circulantes dans le sang estimé à une cellule par millilitre de sang, soit une cellule foetale pour 10 millions de leucocytes, l'enrichissement obtenu est d'un facteur d'environ 6,6 millions (une cellule foetale pour 1,5 cellules larges retenues sur le filtre). De plus, cette approche permet d'obtenir des informations morphologiques sur les cellules foetales circulantes dans le sang. Des cellules mononucléées qui sont probablement des cellules de type cytotrophoblaste, et les cellules polynucléées qui sont les cellules syncytiotrophoblastiques ont été isolées. Ces deux types de cellules n'ont jamais été observés chez 22 donneurs de sang sains et 44 patients avec carcinome analysés selon la même technique de séparation et de microdissection (résultats non présentés).

En outre, les progéniteurs lymphoïdes ou myéloïdes foetaux persistent dans la circulation sanguine après la grossesse, mais non les cellules trophoblastiques. L'avantage de l'analyse des cellules trophoblastiques est donc aussi qu'elle permet d'associer le résultat du test génétique de façon certaine à la grossesse en cours.

Un autre avantage de cette approche est la possibilité de réaliser au moins 5 analyses PCR à partir de l'ADN d'une seule cellule individuelle. Cela donne la possibilité de réaliser un test génétique sur les cellules dont l'origine foetale aura été prouvée en parallèle par l'analyse moléculaire, en particulier par l'analyse de marqueurs polymorphes STR. Il est aussi possible de fonder le diagnostic sur la confrontation de résultats indépendants obtenus sur différentes cellules foetales individuelles isolées de prélèvements répétés d'échantillon de sang. De manière intéressante, on remarquera que le protocole FISH peut aussi être appliqué avec succès à des cellules isolées selon la méthode de filtration (9). Les autres avantages de la méthode selon l'invention résident, d'une part, en sa sensibilité puisque près de 100% des cellules foetales collectées sur la base de leur observation morphologique et analysées sont identifiées comme telles par le procédé de l'invention, et d'autre part, en sa spécificité puisque, en particulier les cellules apoptotiques foetales et les cellules d'origine maternelle peuvent être soustraites à l'analyse au vu de leurs caractéristiques génétiques et morphologiques.

La méthode de l'invention propose donc une nouvelle approche pour la réalisation d'un diagnostic prénatal précoce, non-invasive et particulièrement sensible et spécifique.

### BIBLIOGRAPHIE

1. Bianchi D. Fetal cells in the maternal circulation: feasibility for prenatal diagnosis. Br J Haematol 1999 105: 574-583
2. Fisk N. Maternal-fetal medicine and prenatal diagnosis. Curr Opin Obstet Gynecol 1998 10: 81-83
3. Di Naro E, Ghezzi F, Vitucci A, et al. Prenatal diagnosis of β-thalassemia using fetal erythroblasts enriched from maternal blood by a novel gradient. Mol Hum Reprod 2000 6: 571-574
4. Watanabe A, Sekizawa A, Taguchi A, et al. Prenatal diagnosis of ornithine transcarbamylase deficiency by using a single nucleated erythrocyte from maternal blood. Hum Genet 1998 102: 611-615
5. Takabayashi H, Kuwabara S, Ukita T, Ikawa K, Yamafuji K, Igarashi T. Development of non-invasive fetal DNA diagnosis from maternal blood. Prenat Diagn 1995 15: 74-77
6. Sekizawa A, Taguchi A, Watanabe A, et al. Analysis of HLA-DQ alpha sequences for prenatal diagnosis in single fetal cells from maternal blood. Hum Genet 1998 102: 393-396
7. Bianchi DW. Current knowledge about fetal blood cells in the maternal circulation. J Perinat Med 1998 26: 175-85
8. Bianchi DW et al., PNAS 1996, 93 : 705
9. Vona G, Sabile A, Louha M, et al. Isolation by Size of Epithelial Tumor cells. A new method for the immunomorphological and molecular characterization of circulating tumor cells. Am J Pathol 2000 156:57-63.
10. Poon LL, Leung TN, Lau TK, Lo YM. Presence of fetal RNA in maternal plasma [In Process Citation]. Clin Chem 2000; 46(11):1832-4.
11. Lohse J., Dahl O., Nielsen, PE. PNAS 1999 96: 11804-11808. Double duplex invasion by peptide nucleic acid : a general principle for sequence-specific targeing of double-stranded DNA
12. Dib, C., Faure, S., Fizames, C., Samson, D., Drouot, N., Vignal, A., Missasseau, P., Marc, S., Hazan, J., Seboun, E., Lathrop, M., Gyapay, G., Morissette, J., and Weissenbach, J. A comprehensive genetic map of the human genome based on 5.264 microsatelites. Nature 1996 380 : 152-154
13. Zhang, L., Cui, X., Schmitt, K., Hubert, R.WN., Arnheim, M. Whole genomic amplification from a single cell : implications for genetic analysis. PNAS 1992 89 : 5847-5851
14. Sambrook et al. Molecular Cloning : A laboratory Manual. 2001 3rd Ed., Cold Spring Harbor Laboratory Press, Cold Spring Harbor, New York
15. Diagnostics prénatals et biologie moléculaire, F. Forestier et DF Schroderet, 1997 Ed. Tec et Doc Lavoisier.
16. Voullaire L, Wilton L, Slater H and Williamson R. Prenat Diagn 1999 19 : 846-851
17. Lo YM, Patel P; Sampietro M, Gillmer MD, Fleming KA, Wainscoat JS. Detection of single-copy fetal DNA sequence from maternal blood [letter]. Lancet 1990;335: 1463-4.
18. Paterlini P, Gerken G, Nakajima E, et al. Polymerase chain reaction to detect hepatitis B virus DNA and RNA sequences in primary liver cancers from patients negative for hepatitis B surface antigen. N Engl J Med 1009;323:80-85.

## Revendications

1. Méthode de diagnostic prénatal sur cellules foetales isolées du sang maternel comprenant les étapes suivantes :
a. la filtration d'un échantillon de sang maternel pur ou dilué sur un filtre de porosité comprise entre 6 et 15 microns, de façon à concentrer sur le filtre selon leur taille certaines cellules circulantes, et en particulier des cellules d'origine foetale, ayant une taille comprise entre 14 et 47 microns ;
b. l'analyse et la caractérisation des cellules retenues isolées sur le filtre par la recherche de la présence de marqueur(s) immunologique(s) et/ou cytologique(s) et/ou génétique(s), afin d'identifier leur nature épithéliale et d'obtenir une présomption de leur origine foetale ou maternelle ; et la collecte individuelle de la ou des cellules épithéliales dont l'origine foetale est présumée ;
c. la démonstration, par analyse génétique, de l'origine foetale de cellule(s) d'origine foetale présumée, collectée(s) individuellement à l'étape b. ; et,
d. la recherche d'anomalies génétiques sur le(s) génome(s) de cellule(s) dont l'origine foetale a été démontrée à l'étape c.

2. Méthode selon la revendication 1, **caractérisée en ce que** l'on procède à l'identification de l'origine foetale ou maternelle des cellules à l'étape (c) par la recherche de marqueur(s) génétique(s) caractéristique(s) des cellules foetales.

3. Méthode selon l'une quelconque des revendications 1 à 2, **caractérisée en ce que** la pression de filtration appliquée à ladite étape a. est comprise entre 5 kPa (0,05 bar) et 80 kPa (0,8 bar), et de préférence 10 kPa (0,1 bar).

4. Méthode selon la revendication 3, **caractérisée en ce que** des cellules retenues sur le filtre sont collectées individuellement par microdissection.

5. Méthode selon la revendication 4, **caractérisée en ce que** la microdissection consiste à découper au laser la partie de la membrane filtrante sur laquelle est retenue une cellule ou à décoller la cellule à l'aide du laser puis à récupérer la cellule unique collectée dans un tube approprié.

6. Méthode selon la revendication 5, **caractérisée en ce que** l'étape (d) est réalisée sur le génome d'une cellule unique collectée.

7. Méthode selon la revendication 6, **caractérisée en ce que** l'analyse génétique sur le génome d'une cellule unique collectée permet (i) la démonstration de l'origine foetale ou maternelle de ladite cellule et (ii) la recherche d'anomalies génétiques ou chromosomiques du foetus ou d'un génotype particulier de celui-ci si l'origine foetale de ladite cellule est démontrée.

8. Méthode selon la revendication 7, **caractérisée en ce que** la démonstration de l'origine foetale ou maternelle d'une cellule collectée, est réalisée par l'identification sur une préparation d'ADN dérivée du génome de la cellule unique collectée d'un ou plusieurs marqueurs génétiques ou de polymorphisme, d'une combinaison de ces marqueurs ou d'un dosage allélique particulier de ces marqueurs, démontrant la contribution bi-parentale à l'ADN foetal.

9. Méthode selon l'une des revendications 7 ou 8, **caractérisée en ce que** la recherche d'une anomalie génétique ou chromosomique du foetus ou d'un génotype particulier de celui-ci est réalisée par l'identification d'une cible génétique sur une préparation d'ADN dérivée du génome de la cellule unique collectée.

10. Méthode selon l'une quelconque des revendications 7 à 9, **caractérisée en ce que**, préalablement à la démonstration de l'origine foetale ou maternelle d'une cellule unique collectée et/ou à la recherche d'une anomalie génétique ou chromosomique du foetus ou d'un génotype particulier de celui-ci, ladite cellule collectée est lysée et l'ensemble de son génome est pré-amplifié et purifié de manière à obtenir une préparation d'ADN pré-amplifié dérivée du génome d'une cellule unique collectée.

11. Méthode selon la revendication 10, **caractérisée en ce que** l'on procède à la démonstration de l'origine foetale ou maternelle d'une cellule collectée puis à la recherche d'une anomalie génétique ou chromosomique du foetus ou d'un génotype particulier de celui-ci par l'amplification de marqueurs génétiques ou de polymorphisme ou d'une combinaison de ces marqueurs ou d'une ou plusieurs séquence(s) portant la ou les cible(s) génétique(s) recherchée(s), à partir de la préparation d'ADN pré-amplifié dérivée du génome de ladite cellule.

12. Méthode selon la revendication 11, **caractérisée en ce que** l'amplification d'au moins un marqueur génétique ou de polymorphisme ou d'au moins une séquence portant une cible génétique, est réalisée à partir de moins d'un cinquième de la préparation d'ADN pré-amplifié.

13. Méthode selon l'une des revendications 11 ou 12, **caractérisée en ce que** l'on procède à la démonstration de l'origine foetale ou maternelle d'une cellule collectée et/ou à la recherche d'une anomalie génétique ou chromosomique du foetus ou d'un génotype particulier de celui-ci par le séquençage des marqueurs ou cibles génétiques amplifiés.

14. Méthode selon la revendication 10, **caractérisée en ce que** l'on procède à la démonstration de l'origine foetale ou maternelle d'une cellule collectée et/ou à la recherche d'une anomalie génétique ou chromosomique du foetus ou d'un génotype particulier de celui-ci par l'hybridation de tout ou partie de la préparation d'ADN pré-amplifié avec des sondes d'ADN spécifiques ou de type PNA (Peptide Nucleic Acid).

15. Méthode selon la revendication 14, **caractérisée en ce que** les sondes d'ADN spécifiques sont fixées sur un support formant une puce à ADN ou micro-réseaux ou macro-réseaux.

16. Méthode selon l'une quelconque des revendications 9 à 15, **caractérisée en ce qu'**au moins un marqueur de polymorphisme à identifier est un marqueur microsatellite, un marqueur VNTR (Variable Number of Tandem Repeats), un marqueur SNP (Single Nucleotide Polymorphism) ou un marqueur STR (Short Tandem Repeats).

17. Méthode selon l'une quelconque des revendications 9 à 16, **caractérisée en ce que** l'on procède à la démonstration de l'origine foetale ou maternelle d'une cellule collectée par la recherche d'un marqueur ou d'une combinaison de marqueurs ou d'un dosage allélique de ces marqueurs se distinguant de ceux détectés sur le génome de cellules non maternelles, en particulier par la recherche sur le génome de ladite cellule collectée, d'un marqueur ou d'une combinaison de marqueurs spécifique de l'ADN de cellules paternelles.

18. Méthode selon la revendication 10, **caractérisée en ce que** l'on procède à la recherche d'une anomalie chromosomique par une méthode d'hybridation génomique comparative (CGH) d'une préparation d'ADN pré-amplifié dérivée du génome d'une cellule unique collectée et dont l'origine foetale est démontrée, et d'une préparation d'ADN pré-amplifié de cellules d'origine maternelle ou de cellules de référence non foetales.

19. Méthode selon la revendication 1, **caractérisée en ce que** l'on procède au diagnostic prénatal d'une anomalie chromosomique ou du sexe du foetus, par l'hybridation *in situ* d'une sonde spécifique de l'anomalie chromosomique ou du sexe à détecter, sur le génome des cellules retenues sur le filtre.

20. Méthode selon l'une quelconque des revendications 1 à 19, **caractérisée en ce que** l'échantillon de sang maternel filtré est issu d'un prélèvement de sang effectué après la 5ème semaine de grossesse.

21. Méthode selon l'une quelconque des revendications 1 à 20, **caractérisée en ce que** l'analyse génétique des cellules retenues sur le filtre est réalisée à partir de la filtration de 1 à 10 ml de sang maternel prélevé.

22. Méthode selon l'une quelconque des revendications 1 à 21, **caractérisée en ce que** l'échantillon de sang maternel prélevé est dilué de 10 à 100 fois dans une solution de filtration.

23. Méthode selon l'une quelconque des revendications précédentes, **caractérisée en ce que** l'on procède à la filtration de l'échantillon de sang maternel pur ou dilué à l'aide d'un filtre dont les pores ont un diamètre d'environ 8 µm.

24. Méthode selon la revendication 23, **caractérisée en ce que** le filtre présente des pores d'un diamètre d'environ 8 µm et une densité de pores comprise entre 5.10⁴ et 5.10⁵ pores/cm².

25. Méthode selon l'une quelconque des revendications 1 à 24, **caractérisée en ce que** le filtre est une membrane filtrante de polycarbonate dont la taille des pores est calibrée.

26. Méthode selon l'une quelconque des revendications 1 à 25, **caractérisée en ce que** ladite recherche de la présence de marqueur(s) immunologique(s) comprend un immunomarquage à l'aide d'un anticorps anti-cytokeratines.

27. Méthode selon l'une quelconque des revendications 1 à 26, **caractérisée en ce que** ladite recherche de la présence de marqueur(s) immunologique(s) comprend la recherche de la présence d'antigènes associés aux cellules trophoblastiques.

28. Méthode selon l'une quelconque des revendications 1 à 27, **caractérisée en ce que** ladite recherche de la présence de marqueur(s) cytologique(s) comprend la recherche de la présence de marqueurs cytologiques spécifiques de cellules cytotrophoblastiques et/ou syncyciotrophoblastiques.

29. Méthode selon l'une quelconque des revendications 1 à 28, **caractérisée en ce que** ledit sang maternel est le sang d'une femme enceinte entre la 5^{ème} et la 15^{ème} semaine de grossesse.

30. Méthode selon l'une quelconque des revendications 1 à 29, **caractérisée en ce qu'**avant ladite étape de filtration, ledit échantillon de sang maternel est dilué dans une solution de filtration consistant en un réactif de fixation des cellules nucléées et/ou de lyse des globules rouges.

31. Méthode selon l'une quelconque des revendications 1 à 29, **caractérisée en ce que** les cellules retenues sur le filtre sont remises en culture pour l'obtention d'une culture de cellules concentrées en cellules foetales.

## Patentansprüche

1. Verfahren zur Pränataldiagnostik an fetalen Zellen, die aus dem Blut der Mutter isoliert wurden, das die folgenden Schritte umfasst:
a. Filtration einer Probe unverdünnten oder verdünnten mütterlichen Bluts auf einem Filter mit einer Porosität zwischen 6 und 15 Mikrometer, derart, dass bestimmte zirkulierende Zellen, insbesondere Zellen fetaler Herkunft, mit einer Größe zwischen 14 und 47 Mikrometer entsprechend ihrer Größe auf dem Filter konzentriert werden;
b. Untersuchung und Kennzeichnung der auf dem Filter zurückgehaltenen isolierten Zellen durch Suche nach dem Vorliegen eines oder mehrerer immunologischer und/oder zytologischer und/oder genetischer Marker, zur Identifizierung ihrer Epithelart und zum Erlangen einer Vermutung über ihre fetale oder mütterliche Herkunft; und einzelne Entnahme der Epithelzelle(n), deren fetale Herkunft vermutet wird;
c. Nachweis der fetalen Herkunft einer oder mehrerer in Schritt b. einzeln entnommenen Zellen mit vermuteter fetaler Herkunft durch genetische Untersuchung; und
d. Suche nach genetischen Anomalien an dem (den) Genom(en) der Zelle(n), deren fetale Herkunft in Schritt c. nachgewiesen wurde.

2. Verfahren nach Anspruch ,1
**dadurch gekennzeichnet, dass** die Identifizierung der fetalen oder mütterlichen Herkunft der Zellen in Schritt (c) durch die Suche nach einem oder mehreren für fetale Zellen charakteristischen genetischen Markern erfolgt.

3. Verfahren nach einem der Ansprüche 1 bis 2,
**dadurch gekennzeichnet, dass** der in Schritt a. angelegte Filtrationsdruck zwischen 5 kPa (0,05 bar) und 80 kPa (0,8 bar) und vorzugsweise bei 10 kPa (0,1 bar) liegt.

4. Verfahren nach Anspruch 3,
**dadurch gekennzeichnet, dass** die auf dem Filter zurückgehaltenen Zellen durch Mikrodissektion einzeln entnommen werden.

5. Verfahren nach Anspruch 4,
**dadurch gekennzeichnet, dass** die Mikrodissektion darin besteht, mit dem Laser den Teil der Filtermembran, auf der eine Zelle zurückgehalten wird, abzutrennen oder die Zelle mithilfe des Lasers abzulösen und anschließend die entnommene einzelne Zelle in einem geeigneten Röhrchen zu sammeln.

6. Verfahren nach Anspruch 5,
**dadurch gekennzeichnet, dass** Schritt (d) am Genom einer entnommenen einzelnen Zelle durchgeführt wird.

7. Verfahren nach Anspruch 6,
**dadurch gekennzeichnet, dass** die genetische Untersuchung am Genom einer entnommenen einzelnen Zelle (i) den Nachweis der fetalen oder mütterlichen Herkunft der Zelle und (ii) die Suche nach Gen- oder Chromosomenanomalien des Fetus oder einem speziellen Genotyp von diesem erlaubt, wenn die fetale Herkunft der Zelle nachgewiesen ist.

8. Verfahren nach Anspruch 7,
**dadurch gekennzeichnet, dass** der Nachweis der fetalen oder mütterlichen Herkunft einer entnommenen Zelle durch die Identifizierung eines oder mehrerer genetischer oder polymorpher Marker, einer Kombination dieser Marker oder einer speziellen Alleldosierung dieser Marker auf einer DNA-Präparation aus dem Genom der entnommenen einzelnen Zelle erfolgt, wodurch der biparentale Beitrag zur fetalen DNA nachgewiesen wird.

9. Verfahren nach einem der Ansprüche 7 oder 8,
**dadurch gekennzeichnet, dass** die Suche nach einer Gen- oder Chromosomenanomalie des Fetus oder einem speziellen Genotyp von diesem durch die Identifizierung eines Genziels auf einer DNA-Präparation aus einem Genom der entnommenen einzelnen Zelle erfolgt.

10. Verfahren nach einem der Ansprüche 7 bis 9,
**dadurch gekennzeichnet, dass** vor dem Nachweis der fetalen oder mütterlichen Herkunft einer entnommenen einzelnen Zelle und/oder der Suche nach einer Gen- oder Chromosomenanomalie des Fetus oder einem speziellen Genotyp von diesem die entnommene Zelle lysiert wird und ihr gesamtes Genom präamplifiziert und gereinigt wird, derart, dass man eine Präparation präamplifizierter DNA aus dem Genom einer entnommenen einzelnen Zelle erhält.

11. Verfahren nach Anspruch 10,
**dadurch gekennzeichnet, dass** der Nachweis der fetalen oder mütterlichen Herkunft einer entnommenen Zelle und anschließend die Suche nach einer Gen- oder Chromosomenanomalie des Fetus oder einem speziellen Genotyp von diesem durch Amplifikation genetischer oder polymorpher Marker oder einer Kombination dieser Marker oder einer oder mehrerer das oder die gesuchten Genziele tragenden Sequenzen mithilfe der Präparation präamplifizierter DNA aus dem Genom dieser Zelle erfolgt.

12. Verfahren nach Anspruch 11,
**dadurch gekennzeichnet, dass** die Amplifikation wenigstens eines genetischen oder polymorphen Markers oder wenigstens einer ein Genziel tragenden Sequenz mithilfe wenigstens eines Fünftel der Präparation präamplifizierter DNA erfolgt.

13. Verfahren nach einem der Ansprüche 11 oder 12,
**dadurch gekennzeichnet, dass** der Nachweis der fetalen oder mütterlichen Herkunft einer entnommenen Zelle und/oder die Suche nach einer Gen- oder Chromosomenanomalie des Fetus oder einem speziellen Genotyp von diesem durch Sequenzierung der amplifizierten Marker oder Genziele erfolgt.

14. Verfahren nach Anspruch 10,
**dadurch gekennzeichnet, dass** der Nachweis der fetalen oder mütterlichen Herkunft einer entnommenen Zelle und/oder die Suche nach einer Gen- oder Chromosomenanomalie des Fetus oder einem speziellen Genotyp von diesem durch Hybridisierung der gesamten oder von Teilen der Präparation präamplifizierter DNA mit spezifischen DNA-Sonden oder PNA-Sonden (Peptidnucleinsäure) erfolgt.

15. Verfahren nach Anspruch 14,
**dadurch gekennzeichnet, dass** die spezifischen DNA-Sonden auf einem Träger befestigt sind, der einen DNA-Chip oder Mikroarrays oder Makroarrays bildet.

16. Verfahren nach einem der Ansprüche 9 bis 15,
**dadurch gekennzeichnet, dass** wenigstens ein zu identifizierender polymorpher Marker ein Mikrosatellitenmarker, ein VNTR-Marker (Variable Number of Tandem Repeats), ein SNP-Marker (Single Nucleotide Polymorphism) oder ein STR-Marker (Short Tandem Repeats) ist.

17. Verfahren nach einem der Ansprüche 9 bis 16,
**dadurch gekennzeichnet, dass** der Nachweis der fetalen oder mütterlichen Herkunft einer entnommenen Zelle durch die Suche nach einem Marker oder einer Kombination von Markern oder einer Alleldosierung dieser Marker, die sich von den auf dem Genom nicht mütterlicher Zellen detektierten unterscheiden, erfolgt, insbesondere durch die Suche nach einem Marker oder einer Kombination von Markern auf dem Genom der entnommenen Zelle, die für die DNA paternaler Zellen spezifisch sind.

18. Verfahren nach Anspruch 10,
**dadurch gekennzeichnet, dass** die Suche nach einer Chromosomenanomalie mittels einer Methode zur vergleichenden genomischen Hybridisierung (CGH) einer Präparation präamplifizierter DNA aus dem Genom einer entnommenen einzelnen Zelle, deren fetale Herkunft nachgewiesen ist, und einer Präparation präamplifizierter DNA von Zellen mütterlicher Herkunft oder nicht fetaler Referenzzellen erfolgt.

19. Verfahren nach Anspruch 1,
**dadurch gekennzeichnet, dass** die Pränataldiagnostik einer Chromosomenanomalie oder des Geschlechts des Fetus durch die In-situ-Hybridisierung einer für die zu detektierende Chromosomenanomalie oder das zu detektierende Geschlecht spezifischen Sonde an dem Genom der auf dem Filter zurückgehaltenen Zellen erfolgt.

20. Verfahren nach einem der Ansprüche 1 bis 19,
**dadurch gekennzeichnet, dass** die gefilterte Blutprobe der Mutter aus einer nach der 5. Schwangerschaftswoche erfolgten Blutentnahme stammt.

21. Verfahren nach einem der Ansprüche 1 bis 20,
**dadurch gekennzeichnet, dass** die genetische Untersuchung der auf dem Filter zurückgehaltenen Zellen mittels Filtration von 1 bis 10 ml entnommenen mütterlichen Bluts erfolgt.

22. Verfahren nach einem der Ansprüche 1 bis 21,
**dadurch gekennzeichnet, dass** die Blutprobe der Mutter in einer Filtrationslösung 10 bis 100-fach verdünnt wird.

23. Verfahren nach einem der vorhergehenden Ansprüche,
**dadurch gekennzeichnet, dass** die Filtration der unverdünnten oder verdünnten Blutprobe der Mutter mithilfe eines Filters erfolgt, dessen Poren einen Durchmesser von ca. 8 µm haben.

24. Verfahren nach Anspruch 23,
**dadurch gekennzeichnet, dass** der Filter Poren mit einem Durchmesser von ca. 8 µm und einer Porendichte zwischen 5.10⁴ und 5.10⁵ Poren /cm² aufweist.

25. Verfahren nach einem der Ansprüche 1 bis 24,
**dadurch gekennzeichnet, dass** der Filter eine Filtermembran aus Polycarbonat ist, deren Porengröße kalibriert ist.

26. Verfahren nach einem der Ansprüche 1 bis 25,
**dadurch gekennzeichnet, dass** die Suche nach dem Vorliegen eines oder mehrerer immunologischer Marker eine Immunmarkierung mithilfe eines Anti-Cytokeratin-Antikörpers umfasst.

27. Verfahren nach einem der Ansprüche 1 bis 26,
**dadurch gekennzeichnet, dass** die Suche nach dem Vorliegen eines oder mehrerer immunologischer Marker die Suche nach dem Vorliegen von Trophoblastzellen zugeordneten Antigenen umfasst.

28. Verfahren nach einem der Ansprüche 1 bis 27,
**dadurch gekennzeichnet, dass** die Suche nach dem Vorliegen eines oder mehrerer zytologischer Marker die Suche nach dem Vorliegen zytologischer Marker umfasst, die für Zytotrophoblastzellen und/oder Synzytiotrophoblastzellen spezifisch sind.

29. Verfahren nach einem der Ansprüche 1 bis 28,
**dadurch gekennzeichnet, dass** es sich bei dem Blut der Mutter um Blut einer in der 5. bis 15. Schwangerschaftswoche schwangeren Frau handelt.

30. Verfahren nach einem der Ansprüche 1 bis 29,
**dadurch gekennzeichnet, dass** vor dem Filtrationsschritt die Blutprobe der Mutter in einer Filtrationslösung verdünnt wird, die aus einem Reagens zur Fixierung kernhaltiger Zellen und/oder zur Lyse roter Blutkörperchen besteht.

31. Verfahren nach einem der Ansprüche 1 bis 29,
**dadurch gekennzeichnet, dass** die auf dem Filter zurückgehaltenen Zellen erneut kultiviert werden, um eine mit fetalen Zellen angereicherte Zellkultur zur erhalten.

## Claims

1. A method for prenatal diagnosis of foetal cells isolated from maternal blood, comprising the following steps:
a) filtering a sample of pure or diluted maternal blood on a filter with a pore size in the range 6 to 15 microns, so as to concentrate certain circulating cells on the filter according to their size, and in particular cells of foetal origin with a size in the range 14 to 47 microns;
b) analysing and characterizing the isolated cells retained on the filter by investigating the presence of immunological and/or cytological and/or genetic marker(s) in order to identify their epithelial nature and to obtain a presumption of their foetal or maternal origin; and individually collecting the epithelial cell or cells the foetal origin of which is presumed;
c) demonstrating the foetal origin of the cell(s) of presumed foetal origin individually collected in step b), by genetic analysis;
d) investigating genetic anomalies on the genome(s) of cell(s) the foetal origin of which has been demonstrated in step c).

2. A method according to claim 1, **characterized in that** the foetal or maternal origin of the cells in step c) is identified by investigating genetic marker(s) characteristic of foetal cells.

3. A method according to claim 1 or claim 2, **characterized in that** the filtration pressure applied in said step a) is in the range 5 kPa (0.05 bar) to 80 kPa (0.8 bar), preferably 10 kPa (0.1 bar).

4. A method according to claim 3, **characterized in that** the cells retained on the filter are collected individually by microdissection.

5. A method according to claim 4, **characterized in that** the microdissection consists of laser cutting the portion of the filtration membrane on which a cell is retained or detaching the cell using a laser then recovering the single collected cell in a suitable tube.

6. A method according to claim 5, **characterized in that** step d) is carried out on the genome of a single collected cell.

7. A method according to claim 6, **characterized in that** the genetic analysis of the genome of a single collected cell can (i) demonstrate the foetal or maternal origin of said cell and (ii) allow the search for genetic or chromosomal anomalies of the foetus or of a particular genotype thereof if the foetal origin of said cell is demonstrated.

8. A method according to claim 7, **characterized in that** the foetal or maternal origin of a collected cell is demonstrated by the identification, on a DNA preparation derived from the genome of the single collected cell, of one or more genetic markers or of polymorphism, of a combination of said markers or of a particular allele assay of said markers, demonstrating the biparental contribution of the foetal DNA.

9. A method according to claim 7 or claim 8, **characterized in that** a genetic or chromosomal anomaly of the foetus or of a particular genotype thereof is investigated by identifying a genetic target on a preparation of DNA derived from the genome of the single collected cell.

10. A method according to any one of claims 7 to 9 **characterized in that**, prior to demonstrating the foetal or maternal origin of a single collected cell and/or searching for a genetic or chromosomal anomaly of the foetus or of a particular genotype thereof, said collected cell is lysed and its entire genome is preamplified and purified in order to obtain a preparation of preamplified DNA derived from the genome of a single collected cell.

11. A method according to claim 10, **characterized in that** the foetal or maternal origin of a collected cell is demonstrated then a genetic or chromosomal anomaly of the foetus or of a particular genotype thereof is investigated by amplification of genetic markers or of polymorphism or of a combination of said markers or one or more sequence(s) carrying the identified genetic target(s), starting from the preamplified DNA preparation derived from the genome of said cell.

12. A method according to claim 11, **characterized in that** amplification of at least one genetic marker or of polymorphism or of at least one sequence carrying a genetic target is carried out starting from less than one fifth of the preamplified DNA preparation.

13. A method according to claim 11 or claim 12, **characterized in that** the foetal or maternal origin of a collected cell is demonstrated and/or a genetic or chromosomal anomaly of a foetus or of a particular genotype thereof is investigated by sequencing amplified genetic targets or markers.

14. A method according to claim 10, **characterized in that** the foetal or maternal origin of a collected cell is demonstrated and/or a genetic or chromosomal anomaly of a foetus or of a particular genotype thereof is investigated by hybridization of all or a portion of the preamplified DNA preparation with specific DNA probes or PNA (Peptide Nucleic Acid) type probes.

15. A method according to claim 14, **characterized in that** the specific DNA probes are fixed on a support forming a DNA micro-array or macro-array chip.

16. A method according to any one of claims 9 to 15, **characterized in that** at least one polymorphism marker to be identified is a microsatellite marker, a VNTR (Variable Number of Tandem Repeats) marker, a SNP (Single Nucleotide Polymorphism) marker or a STR (Short Tandem Repeat) marker.

17. A method according to any one of claims 9 to 16, **characterized in that** the foetal or maternal origin of a collected cell is demonstrated by searching for a marker or a combination of markers or by an allele assay of said markers distinguished from those detected on the genome of non-maternal cells, in particular by searching for a marker or a combination of markers specific to the DNA of paternal cells on the genome of said collected cell.

18. A method according to claim 10, **characterized in that** a chromosomal anomaly is identified by a method for comparative genomic hybridization (CGH) of a preamplified DNA preparation derived from the genome of a single collected cell and the foetal origin of which has been demonstrated, and of a preamplified DNA preparation of cells of maternal origin or non-foetal reference cells.

19. A method according to claim 1, **characterized in that** prenatal diagnosis of a chromosomal anomaly or the gender of a foetus is carried out by in situ hybridization of a specific probe for a chromosomal anomaly or the gender to be detected on the genome of cells retained on the filter.

20. A method according to any one of claims 1 to 19, **characterized in that** the filtered maternal blood is derived from a blood sample taken after the fifth week of pregnancy.

21. A method according to any one of claims 1 to 20, **characterized in that** the genetic analysis of cells retained on the filter is carried out by filtering 1 to 10 mL of maternal blood.

22. A method according to any one of claims 1 to 21, **characterized in that** the maternal blood sample is diluted 10- to 100-fold in a filtration solution.

23. A method according to any one of the preceding claims, **characterized in that** the pure or diluted maternal blood sample is filtered using a filter with a pore size having a diameter of approximately 8 µm.

24. A method according to claim 23, **characterized in that** the filter has pores with a diameter of approximately 8 µm and a pore density in the range 5 x 10⁴ to 5 x 10⁵ pores/cm².

25. A method according to any one of claims 1 to 24, **characterized in that** the filter is a polycarbonate filtration membrane the pore size of which is graded.

26. A method according to any one of claims 1 to 25, **characterized in that** said investigation of the presence of immunological marker(s) comprises immunolabelling with the aid of an anti-cytokeratin antibody.

27. A method according to any one of claims 1 to 26, **characterized in that** said investigation of the presence of immunological marker(s) comprises investigating the presence of antigens associated with trophoblastic cells.

28. A method according to any one of claims 1 to 27, **characterized in that** said investigation of the presence of cytological marker(s) comprises investigating the presence of specific cytological markers of cytotrophoblastic and/or syncytiotrophoblastic cells.

29. A method according to any one of claims 1 to 28, **characterized in that** said maternal blood is blood from a pregnant woman between the 5^{th} and 15^{th} week of pregnancy.

30. A method according to any one of claims 1 to 29, **characterized in that** before said filtration step, said sample of maternal blood is diluted in a filtration solution consisting of a reagent for fixing nucleated cells and/or for lysing red globules.

31. A method according to any one of claims 1 to 29, **characterized in that** the cells retained on the filter are cultured in order to obtain a culture of cells concentrated in foetal cells.
